(19) Europäisches Patentamt
European Patent Office
Office européen des brevets

(11) EP 2 263 647 A1

(12) DEMANDE DE BREVET EUROPEEN

(43) Date de publication:
**22.12.2010 Bulletin 2010/51**

(21) Numéro de dépôt: **10166438.1**

(22) Date de dépôt: **18.06.2010**

(51) Int Cl.:
***A61K 8/81*** *(2006.01)* ***A61K 8/90*** *(2006.01)*
***A61Q 5/06*** *(2006.01)* ***A61Q 1/00*** *(2006.01)*

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**
Etats d'extension désignés:
**BA ME RS**

(30) Priorité: **18.06.2009 FR 0954105**

(71) Demandeur: **L'Oréal**
**75008 Paris (FR)**

(72) Inventeur: **Abbas, Karima**
**93200, Saint Denis (FR)**

(74) Mandataire: **Fevrier, Murielle Françoise E.**
**L'Oréal**
**River Plaza - DIPI**
**25-29 Quai Aulagnier**
**92665 Asnières-sur-Seine (FR)**

# (54) Composition de traitement des fibres kératiniques comprenant un polymère supramoéculaire a base de polyalcène et un polymere sequence et un solvant volatil

(57) La présente invention a pour objet une composition de traitement des fibres kératiniques comprenant

- un ou plusieurs polymères supramoléculaires à base de polyalcène,
- un ou plusieurs un ou plusieurs copolymères éthyléniques séquencés, contenant au moins une première séquence ayant une température de transition vitreuse (Tg) supérieure ou égale à 40°C et étant issue en totalité ou en partie de un ou plusieurs premiers monomères, qui sont tels que l'homopolymère préparé à partir de ces monomères a une température de transition vitreuse supérieure ou égale à 40°C, et au moins une deuxième séquence ayant une température de transition vitreuse inférieure ou égale à 20°C et étant issue en totalité ou en partie de un ou plusieurs deuxièmes monomères, qui sont tels que l'homopolymère préparé à partir de ces monomères a une température de transition vitreuse inférieure ou égale à 20°C, ladite première séquence et ladite deuxième séquence étant reliées entre elles par un segment intermédiaire statistique comprenant au moins un desdits premiers monomères constitutifs de la première séquence et au moins un desdits deuxièmes monomères constitutifs de la deuxième séquence, et ledit copolymère séquence ayant un indice de polydispersité 1 supérieur à 2, et
- un ou plusieurs solvants volatils.

La présente invention permet d'obtenir sur les fibres kératiniques des gainages pouvant être colorés permettant de conserver des cheveux individualisés tout en préservant les qualités physiques de la fibre.

EP 2 263 647 A1

## Description

La présente invention a pour objet une composition de traitement des fibres kératiniques, et en particulier des fibres kératiniques humaines telles que les cheveux ainsi qu'une composition de coloration des cheveux à partir d'un pigment.

Les cheveux sont généralement abîmés et fragilisés par l'action des agents atmosphériques extérieurs tels que la lumière et les intempéries, et par des traitements mécaniques ou chimiques tels que le brossage, le peignage, les décolorations, les permanentes et/ou les teintures. Il en résulte que les cheveux sont souvent difficiles à discipliner, en particulier ils sont difficiles à démêler ou à coiffer, et les chevelures, même abondantes, conservent difficilement une coiffure de bon aspect en raison du fait que les cheveux manquent de vigueur, de volume et de nervosité.

Cette dégradation des cheveux est par ailleurs accrue par la répétition de traitement de coloration permanente des cheveux, qui consiste à appliquer sur les cheveux un précurseur de colorant et un agent oxydant.

Ainsi, pour remédier à cela, il est maintenant usuel d'utiliser des produits de coiffage qui permettent de conditionner les cheveux en leur apportant notamment du corps, de la masse ou du volume.

Ces produits de coiffage sont généralement des compositions cosmétiques capillaires comprenant un ou plusieurs polymères qui présentent une forte affinité pour les cheveux et qui ont le plus souvent pour fonction de former un film à leur surface en vue de modifier leurs propriétés superficielles, notamment pour les conditionner.

Un inconvénient lié à l'utilisation de ces compositions capillaires réside dans le fait que les effets cosmétiques conférés par de telles compositions ont tendance à disparaître, notamment dès le premier shampooing.

Il est connu du brevet EP 1 392 222 d'utiliser une composition cosmétique pour le soin et/ou le traitement des matières kératiniques comprenant un polymère supramoléculaire comportant un squelette polymérique et au moins deux groupements capables de former au moins trois liaisons hydrogène, et du brevet EP 1 435 900 d'utiliser une composition capillaire comprenant un polymère supramoléculaire comportant un squelette polymérique et au moins deux groupements capables de former au moins trois liaisons hydrogène et un agent tensio-actif ou un agent de conditionnement des cheveux, ceci pour améliorer la rémanence des gainages.

En particulier, des gainages ont été obtenus à partir de polymère supramoléculaire à base de polyalcène. Lorsque ces polymères sont utilisés sur les cheveux, ces derniers sont gainés de manière homogène tout en restant individualisés. Ce gainage apporte à la chevelure du corps, de la masse de manière rémanente. Lorsque le gainage précédent contient des pigments, la coloration obtenue est chromatique et très visible sur un fond foncé.

Un inconvénient lié aux gainages utilisant un polymère supramoléculaire à base de polyalcène est leur manque de résistance aux agressions extérieures telles que les corps gras et notamment le sébum. La sensibilité du gainage aux corps gras se traduit par une diminution de la tenue du gainage au shampooing et à une dégradation de sa cosmétique, le toucher des cheveux devenant collant.

Ainsi, le but de la présente invention est de fournir une composition de traitement des fibres kératiniques, et en particulier les cheveux, permettant d'obtenir un gainage des cheveux rémanent aux shampoings avec des propriétés améliorées de résistance aux agressions exterieures, notamment du sébum, sans dégradation des fibres kératiniques et tout en conservant des cheveux parfaitement individualisés et non collants.

Ce but est atteint avec la présente invention qui a pour objet une composition de traitement des fibres kératiniques, et en particulier des cheveux comprenant

- un ou plusieurs polymères supramoléculaires à base de polyalcène,
- un ou plusieurs copolymères éthyléniques séquencés, contenant au moins une première séquence ayant une température de transition vitreuse (Tg) supérieure ou égale à 40°C et étant issue en totalité ou en partie de un ou plusieurs premiers monomères, qui sont tels que l'homopolymère préparé à partir de ces monomères a une température de transition vitreuse supérieure ou égale à 40°C, et au moins une deuxième séquence ayant une température de transition vitreuse inférieure ou égale à 20°C et étant issue en totalité ou en partie de un ou plusieurs deuxièmes monomères, qui sont tels que l'homopolymère préparé à partir de ces monomères a une température de transition vitreuse inférieure ou égale à 20°C, ladite première séquence et ladite deuxième séquence étant reliées entre elles par un segment intermédiaire statistique comprenant au moins un desdits premiers monomères constitutifs de la première séquence et au moins un desdits deuxièmes monomères constitutifs de la deuxième séquence, et ledit copolymère séquencé ayant un indice de polydispersité I supérieur à 2, et
- un ou plusieurs solvants volatils.

La composition conforme à la présente invention permet d'obtenir sur les fibres kératiniques des gainages rémanents aux shampooings qui préservent les qualités physiques de la fibre kératinique. Un tel gainage est en particulier résistant aux agressions extérieures que peuvent subir les cheveux telles que le brushing et la transpiration, notamment les corps gras tels que le sébum. Il permet en particulier d'obtenir un dépôt lisse et homogène avec des cheveux parfaitement individualisés, pouvaient être coiffés sans problème.

On entend par cheveux individualisés des cheveux qui après application de la composition et séchage ne sont pas collés (ou sont tous séparés les uns des autres) entre eux et ne forment donc pas des amas de cheveux, le gainage étant formé autour de pratiquement chaque cheveu.

La présente invention a également pour objet un procédé de traitement des fibres kératiniques, et en particulier des cheveux mettant en oeuvre cette composition.

***Polymères supramoléculaires à base de polyalcène***

Au sens de la présente invention, on entend par polymère supramoléculaire à base de polyalcène un polymère comportant dans sa structure au moins une partie polyalcène et au moins une partie comportant au moins un groupe capable de former au moins 3 liaisons H, de préférence 4 liaisons H.

Le polyalcène est choisi de préférence parmi les poly(éthylènebutylène), les polybutadiènes et les polyisoprènes.

Les polymères supramoléculaires de l'invention peuvent être notamment issus de la condensation d'au moins un polymère polyalcène fonctionnalisé par au moins un groupe réactif, avec au moins un greffon fonctionnalisé par au moins un groupe réactif susceptible de réagir avec le ou les groupes réactifs du polymère poly(alcène) fonctionnalisé, le dit greffon étant porteur d'au moins un groupe capable de former au moins 3 liaisons H, de préférence au moins 4 liaisons H.

De préférence, le polymère polyalcène fonctionnalisé est de formule A :

HX-R-X'H

XH et X'H étant des groupes réactifs, avec X et X', identiques ou différents, choisis parmi O, S, NH, ou $NR_a$, $R_a$ représentant un groupe alkyle en $C_1$-$C_6$; de préférence, X et / ou X' désignent O ; encore plus préférentiellement, X et X' désignent O ;

R représente un homo ou un copolymère issu d'un ou plusieurs alcènes mono ou polyinsaturés en $C_2$-$C_{10}$, et de préférence en $C_2$-$C_4$ ; R représente de préférence un poly(éthylènebutylène), un polybutadiène ou un polyisoprène.

Les poly(éthylènebutylène) sont des copolymères de butène 1 et d'éthylène. On peut les schématiser par l'enchaînement de motifs de structures :

$[-CH_2-CH_2-]$ et $[-CH_2CH(CH_2-CH_3)]$

Les polybutadiènes peuvent être des 1,4-polybutadiènes ou des 1,2-polybutabiènes qui peuvent être respectivement schématiser par les enchaînements de motifs suivants :

$[-CH_2-CH=CH-CH_2-]$ (polybutadiènes 1,4)

$[-CH_2-CH(CH=CH_2)-]$ (polybutadiènes 1,2)

De preference, pour les polybutadiènes, il s'agit de polybutadiènes 1,2.

La fonctionnalisation se fait de préférence en bout de chaînes. On parle alors de polymères téléchéliques. Les groupes de fonctionnalisation peuvent être fixés au polymère polyalcène via des linkers, de préférence des groupes alkylène en $C_1$-$C_4$ linéaires ou ramifiés.

Ces poly(alcènes) peuvent être partiellement ou totalement hydrogénés hydrogénés pour limiter ou éviter les risques de réticulations.

Ils peuvent comporter dans leur structure d'autres motifs issus d'autres monomères. Comme comonomères, on peut en particulier citer le styrène.

Les polydiènes, de préférence hydrogénés, à extrémités hydroxyles et les polyoléfines à extrémités hydroxyles sont des squelettes polymériques préférés selon l'invention.

Ces polydiènes à extrémités hydroxyles sont définis par exemple dans le brevet FR 2 782 723 d'ELF ATOCHEM. Ils sont choisis dans le groupe comprenant les homo et copolymères de polybutadiène, de polyisoprène et de poly(1,3-pentadiène). Ce sont des oligomères de masse moléculaire moyenne en nombre inférieure à 7000, et de préférence de 1000 à 5000, présentant une fonctionnalité en extrémités hydroxyles de 1,8 à 3, et de préférence voisine de 2.

On citera en particulier les polybutadiènes hydroxylés commercialisés par la société ELF ATOCHEM sous les marques POLY BD R-45HT et POLY BD R-20 LM, qui seront utilisés de préférence hydrogénés. On peut aussi citer les (1,2- polybutadiènes) hydrogénés di OH tels que le GI3000 de Mn = 2600-3200 et le GI2000 de Mn = 1800-2200 commercialisés par la société Nisso.

On peut également utiliser des polyoléfines, homopolymères ou copolymères, à extrémités $\alpha,\omega$ hydroxyles tels

que :

- les oligomères de polyisobutylène à extrémités $\alpha,\omega$ hydroxyles ;
- les copolymères commercialisés par la société Mitsubishi sous la marque POLYTAIL avec, en particulier, ceux répondant à la formule :

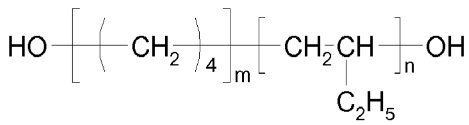

Les polymères supramoléculaires de la présente invention possèdent dans leur structure au moins un greffon étant porteur d'au moins un groupe capable de former au moins 3 liaisons H, de préférence au moins 4 liaisons H.

Ces groupes capables de former au moins 3 liaisons H peuvent comprendre par exemple au moins 3 groupements fonctionnels, de préférence au moins 4, choisis parmi :

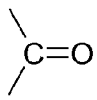 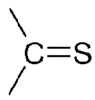 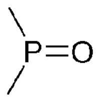 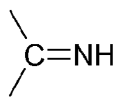 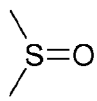

$-C\equiv CH$ -SH -OH 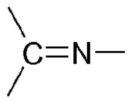 $-C\equiv N$ -NH2

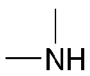 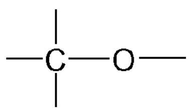 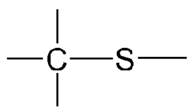 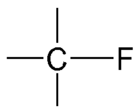

Ces groupements fonctionnels peuvent être classés en deux catégories :

- les groupements fonctionnels donneurs de liaisons H tels que les groupements :

$-C\equiv CH$ -SH -OH 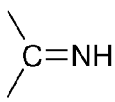 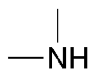 -NH2

- et les groupements fonctionnels accepteurs de liaisons H tels que les groupements :

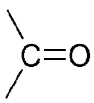 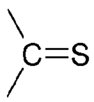 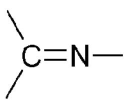 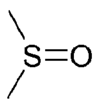

$-C\equiv N$ 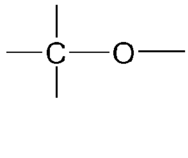 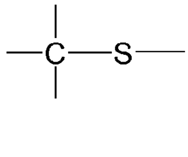 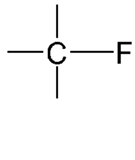

Les groupes capables de former au moins 3 liaisons H forment un élément structural de base comportant au moins 3 groupements, de préférence au moins 4 groupements fonctionnels, et plus préférentiellement 4 groupements fonctionnels, capables d'établir des liaisons H. Les éléments structuraux de base capables d'établir 3 ou 4 liaisons H peuvent être schématisés de la manière suivante :

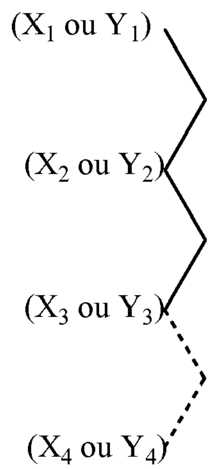

où $X_i$ (i entier naturel) est un groupement fonctionnel accepteur de liaisons H et $Y_i$ est un groupement fonctionnel donneur de liaisons H.

Ainsi, chaque élément structural doit pouvoir établir des liaisons H avec un ou plusieurs éléments structuraux partenaires, identiques (c'est-à-dire auto-complémentaires) ou différents, de telle sorte que chaque appariement de deux éléments structuraux partenaires se fasse par formation d'au moins trois liaisons H, de préférence au moins quatre liaisons H, et plus préférentiellement 4 liaisons H.

Un accepteur de protons X s'appariera avec un donneur de protons Y. Plusieurs possibilités sont offertes, par exemple appariement de :

XXXX avec YYYY ;
XXXY avec YYYX ;
XXYX avec YYXY ;
XYYX avec YXXY ;
XXYY avec YYXX auto-complémentaire ou non ;
XYXY avec YXYX auto-complémentaire ou non.

De préférence, les groupes peuvent établir 4 liaisons H avec un groupe partenaire identique (ou autocomplémentaire) parmi lesquelles 2 liaisons donneur (par exemple NH) et 2 liaisons accepteur (par exemple CO et -C=N-).

De préférence, les groupes capables de former au moins 3 liaisons H comportent des cycles à 5 ou 6 atomes (cycles aromatiques ou hétérocycles insaturés) très souvent constitués d'atomes de C et / ou N et avec des doubles liaisons conjuguées pour stabiliser et diriger les interactions H.

De préférence encore, les groupes capables de former au moins 3 liaisons H sont engagés dans des cycles à 6 atomes comprenant des C et / ou N et avec des doubles liaisons conjuguées pour stabiliser et diriger les interactions H.

Selon un mode de réalisation particulier de l'invention, les groupes capables de former 3 ou 4 liaisons H sont choisis parmi les familles suivantes, étant entendu que toutes les formes tautomériques sont inclues :

- (i) les aminopyrimidones de formule :

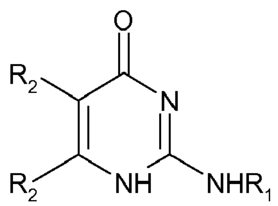

- (ii) les ureïdopyrimidones de formule :

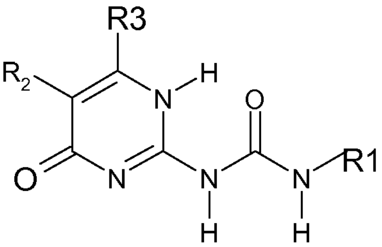

- (iii) les acylaminopyridines et notamment :
- les monoacylaminopyridines de structure :

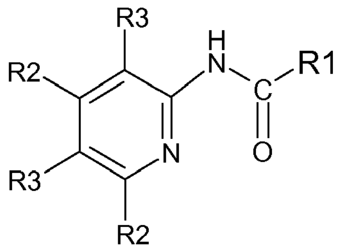

- les di(acylamino)pyridines et plus particulièrement les 2,6-di(acylamino)pyridines de structure :

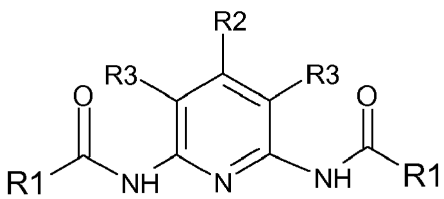

- (iv) les aminopyrimidines, et notamment :
- les composés aminopyrimidines :

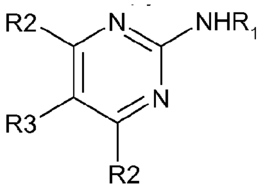 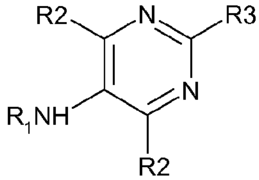 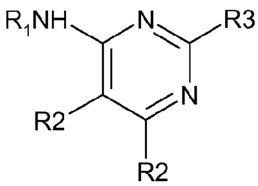

- les composés diaminopyrimidines de structure :

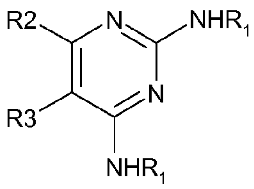 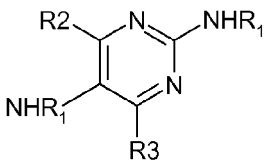 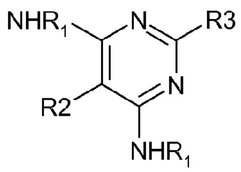 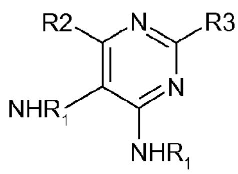 -

les composés triaminopyrimidines ;

- (v) les uréïdotriazines, et notamment les mono-, di- et tri-uréïdotriazines, et en particulier les uréïdoaminotriazines de structure :

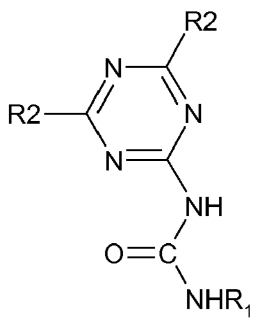

- (vi) les (acylamino)triazines, et notamment les mono-, di- et tri-acylamino triazines, éventuellement amino (mono-, di- ou tri-amino) et en particulier :
- les di(acylamino)triazines de structure :

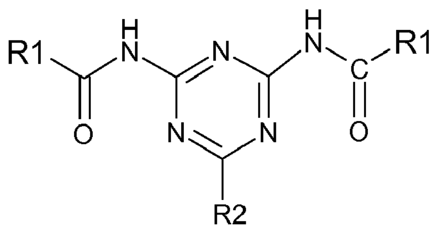

- les acylamino, amino-triazines, (mono ou di- acylamino, et mono- ou di-amino) et notamment les composés de structure :

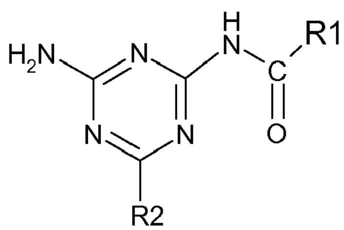

- les acylaminotriazines de structure :

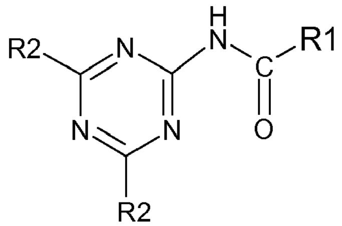

- les tri-acylamino triazines ;
- (vii) les aminotriazines et notamment :
- les monoaminotriazines ;
- les 2,6-diamino-s-triazines de structure :

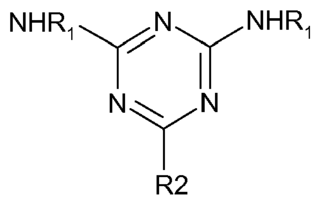

- les composés triamino-s-triazines de structure :

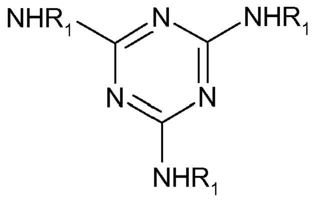

- (viii) les acylaminotriazoles de structure :

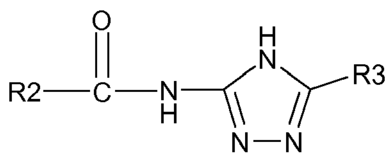

- (ix) les composés de la famille de l'acide urazoyl benzoïque de structure :

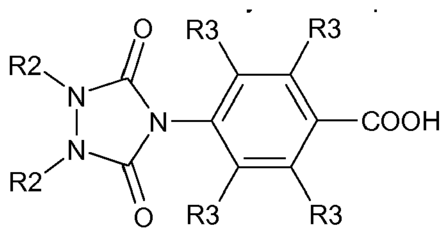

- (x) les phtalhydrazides de structure :

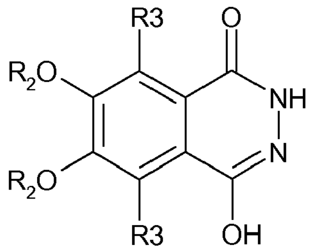

- (xi) les uraciles de structure :

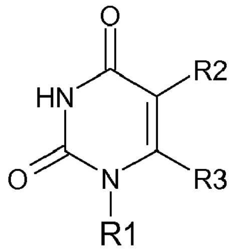

- (xii) les thymines de structure :

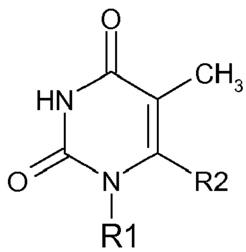

- (xiii) les succinimides de structure :

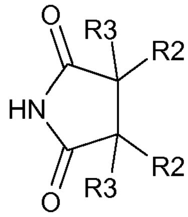

- (xiv) les glutarimides de structure :

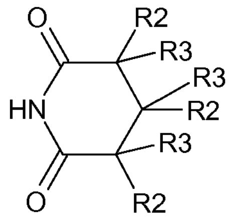

- (xv) les composés de la famille de l'acide cyanurique de structure :

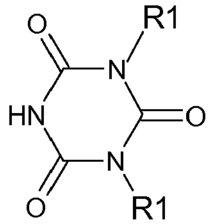

- (xvi) les maléimides :

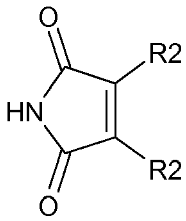

- (xvii) les composés de la famille de l'acide barbiturique, de structure :

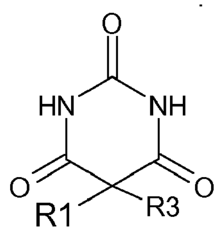

- (xviii) les composés de structure :

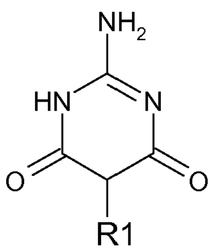

et

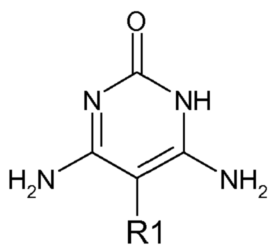

- (xix) les composés de la famille de l'acide triméllitique, de formule :

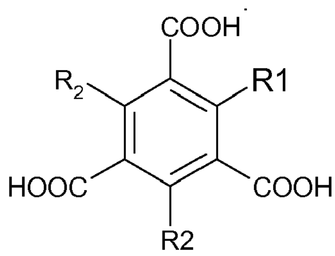

- (xx) les uréïdopyridines, notamment les mono- ou di-uréïdopyridines, et en particulier ceux de formule :

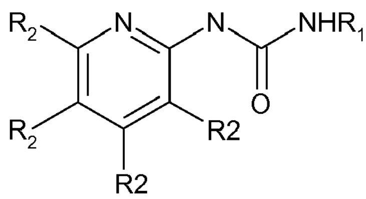

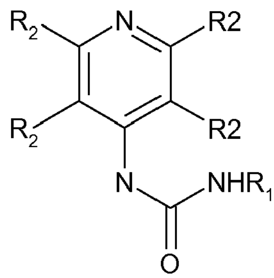

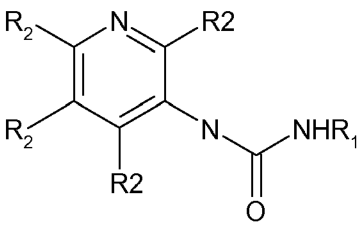

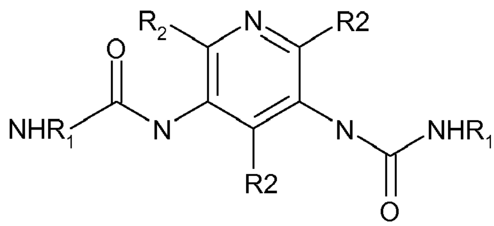

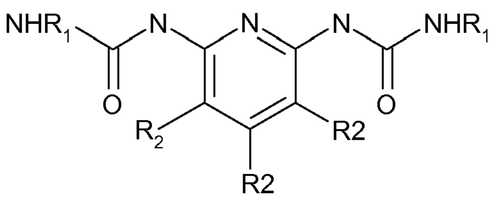

- (xxi) les carbamoylpyridines, de formule :

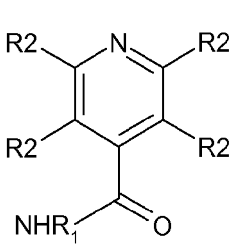

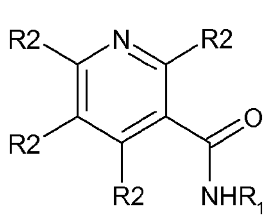

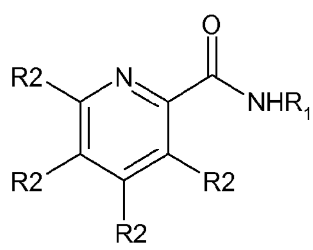

- (xxii) les adénines de formule :

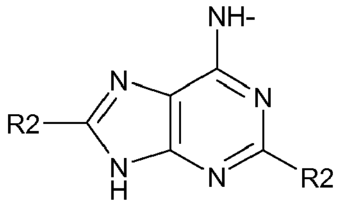

- (xxiii) les guanines de formule :

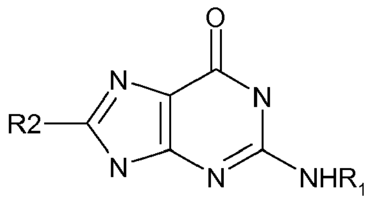

- (xxiv) les cytidines de formule :

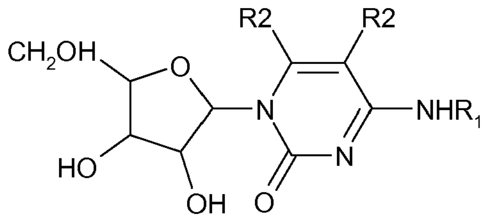

Dans l'ensemble de ces formules, la signification des radicaux est la suivante :

- (a) les radicaux $R_1$, identiques ou différents, représentent une liaison simple, un atome d'hydrogène, un atome d'halogène et / ou un groupe carboné (notamment alkyle) monovalent, linéaire, ramifié ou cyclique, en $C_1$-$C_{6000}$, saturé ou non, éventuellement aromatique, pouvant contenir un ou plusieurs hétéroatomes tels que O, S, N, P, Cl, Br, Cl, Br, F ; ou une combinaison de ces significations.

Le radical $R_1$ peut notamment être un groupe cycloalkyle en $C_4$-$C_{12}$ ; un groupe alkyle linéaire ou ramifié en $C_1$-$C_{30}$ ou un groupe aryle en $C_4$-$C_{12}$ ; éventuellement substitués par une fonction amino, ester et / ou hydroxy.
Le radical $R_1$ peut être aussi un groupement : $C_4H_9$ ; phényle ; 1,4-nitrophényle ; 1,2-éthylène ; 1,6-héxylène ; 1,4-butylène ; 1,6-(2,4,4-triméthylhexylène) ; 1,4-(4-méthylpentylène) ; 1,5-(5-méthylhexylène) ; 1,6-(6-méthylheptylène) ; 1,5-(2,2,5-triméthylhexylène) ; 1,7-(3,7-diméthyloctylène) ; -isophorone- ; 4,4'-méthylène biscyclohexylène ; tolylène ; 2-méthyl-1,3-phénylène ; 4-méthyl-1,3-phénylène ; 4,4-biphénylèneméthylène ;
et de préférence : -isophorone- ; $-(CH_2)_2-$ ; $-(CH_2)_6-$ ; $-CH_2CH(CH_3)-CH_2-C(CH_3)_2-CH_2-CH_2$ ; 4,4'-méthylène biscyclohexylène ; 2-méthyl-1,3-phénylène.
Encore plus préférentiellement, $R_1$ est une liaison simple.

- (b) les radicaux $R_2$, identiques ou différents au sein d'une même formule, représentent une liaison simple, un atome d'hydrogène, un atome d'halogène (-Br, -Cl, -F), un radical -OH, - $N(R)_2$ (avec R étant H ou un radical alkyle, linéaire ou ramifié en $C_1$-$C_{12}$, de préférence en $C_1$-$C_4$, et mieux un radical méthyle ou éthyle) ; ou un groupe hydrocarboné monovalent, linéaire, ramifié ou cyclique, en $C_1$-$C_{6000}$, saturé ou non, éventuellement aromatique, pouvant contenir un ou plusieurs hétéroatomes tels que O, S, N, P, F ; ou une combinaison de ces significations.
  Les radicaux $R_2$ peuvent notamment être H, CN, $NH_2$ ou bien :

  - un groupe alkyle en $C_1$-$C_{30}$ ;
  - un groupe cycloalkyle en $C_4$-$C_{12}$ ;
  - un groupe aryle en $C_4$-$C_{12}$ ;

- un groupe aryl($C_4$-$C_{12}$)alkyle en $C_1$-$C_{30}$ ;
- un groupe alcoxy en $C_1$-$C_4$ ;
- un groupe arylalcoxy, en particulier un groupe aryl($C_1$-$C_4$)alcoxy ;
- un hétérocycle en $C_4$-$C_{12}$ ;
- un groupe thioalcoxy ;
- un groupe sulfoxy ;

ou leurs mélanges, ces groupes étant éventuellement substitués par une fonction amino, ester et / ou hydroxy.

De préférence, $R_2$ représente H, $CH_3$, $C_{13}H_{27}$, $C_7H_{15}$ ou phényle.

- (c) les radicaux $R_3$, identiques ou différents au sein d'une même formule, représentent un atome d'hydrogène ou un groupe hydrocarboné monovalent, linéaire, ramifié ou cyclique, en $C_1$-$C_{6000}$, saturé ou non, éventuellement aromatique, pouvant contenir un ou plusieurs hétéroatomes tels que O, S, N, P, F ; ou une combinaison de ces significations.

Le radical $R_3$ peut notamment être un groupe cycloalkyle en $C_4$-$C_{12}$ ; un groupe alkyle linéaire ou ramifié en $C_1$-$C_{30}$ ou un groupe aryle en $C_4$-$C_{12}$ ; éventuellement substitués par une fonction amino, ester et/ou hydroxy. De préférence, le radical $R_3$ représente un radical méthyle.

Dans l'ensemble de ces formules, il est bien entendu qu'au moins un, notamment un ou deux, des groupes $R_1$ et / ou $R_2$ est une liaison simple constituant le point d'accroche du groupe capable de former au moins 3 liaisons H sur le reste du greffon.

De préférence, ledit point d'accroche est porté par $R_1$ et/ou $R_2$ et de préférence, il est porté par $R_1$.

Les groupes capables de former au moins 3 liaisons H peuvent notamment être choisis parmi :

(a) les groupes capables de former au moins 3 liaisons H complémentaires et identiques c'est-à-dire auto-complémentaires, et notamment :

- les aminopyrimidones, les uréïdopyrimidones,
- les composés de la famille de l'acide triméllitique, ou de l'acide urazoyl benzoïque,
- les acylaminopyridines, les uréïdopyridines, les carbamoylpyridines,
- les acylaminotriazines, les uréidotriazines et notamment les uréïdoaminotriazines, les diamino-triazines,
- les acylaminotriazoles,
- les phtalhydrazides,
- les composés de formule :

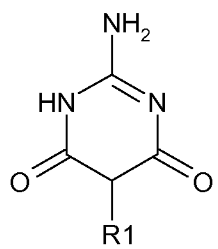

et

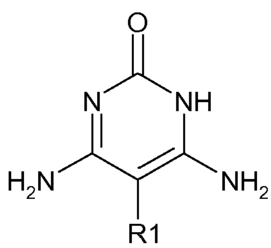

dans lesquelles $R_1$ est un atome d'hydrogène ou un groupe hydrocarboné monovalent, linéaire, ramifié ou cyclique, en $C_1$-$C_{6000}$, saturé ou non, éventuellement aromatique, pouvant contenir un ou plusieurs hétéroatomes tels que O, S, N, P, F.

(b) les groupes capables de former au moins 3 liaisons H complémentaires mais différents, et notamment :

- adénine complémentaire de guanine,
- cytidine complémentaire de thymine,
- triamino-s-triazine complémentaire de uracile ou de succinimide ou de glutarimide ou d'acide cyanurique ou de thymine ou de maléimide ou de (di)aminopyrimidine ou d'acide barbiturique,
- acylamino-amino-s-triazine complémentaire de uracile ou de succinimide ou de glutarimide ou d'acide cyanurique ou de thymine ou de maléimide ou de (di)aminopyrimidine ou d'acide barbiturique.

De manière préférée, les groupes capables de former au moins 3 liaisons H sont choisis parmi les groupes capables d'établir au moins trois liaisons H avec eux-mêmes (auto-complémentaire), notamment au moins quatre liaisons H avec eux-mêmes. Parmi ces groupes, on peut en particulier citer :

- les uréïdopyrimidones ;
- les uréïdopyridines, les carbamoylpyridines ;
- les acylamino-s-triazines et notamment les acyl-diamino-s-triazines ;
- les uréidotriazines ;
- les phtalhydrazides ;
- les composés de formule :

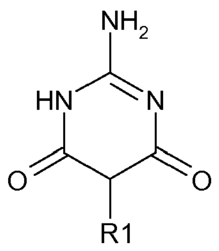

et

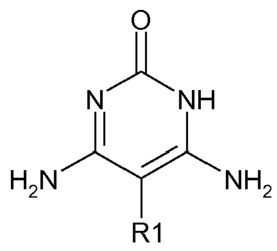

dans lesquels les radicaux $R_1$, $R_2$ et $R_3$ ont les significations données ci-dessus, en particulier les significations données en préférence.

Encore mieux, on peut citer à titre d'exemples préférés de groupes capables de former au moins 3 liaisons H les groupes dérivés des uréidopyrimidones, et en particulier de la 2-uréïdopyrimidone ou de la 6-méthyl, 2-uréïdopyrimidone.

Le reste du greffon est constitué d'un linker L porteur d'au moins un groupe réactif capable de réagir avec le ou les groupe du poly(alcène) fonctionnalisé.

Ce groupe réactif peut être par exemple un groupe carboxy ou un groupe isocyanate. De préférence, il s'agit d'un groupe -N=C=O ou -N=C=S, et encore plus préférentiellement d'un groupe -N=C=O (isocyanate).

De préférence, le linker L est un groupement : phénylène ; 1,4-nitrophényle ; 1,2-éthylène ; 1,6-héxylène ; 1,4-butylène ; 1,6-(2,4,4-triméthylhexylène) ; 1,4-(4-méthylpentylène) ; 1,5-(5-méthylhexylène) ; 1,6-(6-méthylheptylène) ; 1,5-(2,2,5-triméthylhexylène) ; 1,7-(3,7-diméthyloctylène) ; -isophorone-; 4,4'-méthylène biscyclohexylène ; tolylène ; 2-méthyl-1,3-phénylène ; 4-méthyl-1,3-phénylène ; 4,4-biphénylèneméthylène ;
et de préférence : -isophorone- ; $-(CH_2)_2-$ ; $-(CH_2)_6-$ ; $-CH_2CH(CH_3)-CH_2-C(CH_3)_2-CH_2-CH_2$ ; 4,4'-méthylène biscyclohexylène ; 2-méthyl-1,3-phénylène.

Dans une version particulièrement préférée de l'invention, les greffons sont de formule (B) :

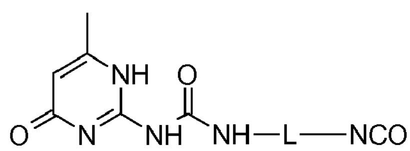

L ayant la même signification que ci-dessus.

Encore plus préférentiellement, le polymère supramoléculaire de l'invention est de formule (C) :

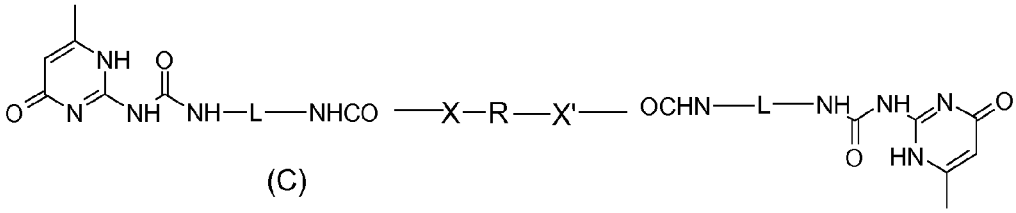

(C)

R, X, X', L ayant les significations indiquées précédemment.

De préférence, dans la formule (C), X et X' désignent un atome d'oxygène.

Le ou les polymères supramoléculaires à base de polyalcène de l'invention peuvent aussi être obtenus à partir d'un polymère (A1) comportant une partie polyalcène, le dit polymère étant fonctionnalisé par au moins un groupe réactif (B1), par condensation avec au moins une molécule (A3) comportant au moins un groupe réactif (B2), la dite molécule étant telle qu'après réaction des groupes (B1) et (B2) il y ait formation d'une entité capable de former au moins 3 liaisons H, de préférence au moins 4 liaisons H.

De préférence, ces entités sont de structures (i) à (xxiv) telles que définies précédemment avec $R_1$ désignant une simple liaison.

Le polymère (A1) peut notamment résulter de l'action sur un polymère polyalcène de formule A telle que définie ci avant de composés (A2) comportant deux groupes réactifs (B'2) capables de réagir avec les groupes fonctionnalisés du polyalcène.

Ces groupes réactifs peuvent être par exemple des groupes carboxy ou des groupes isocyanate. De préférence, il s'agit de groupes -N=C=O ou -N=C=S, et encore plus préférentiellement de groupes -N=C=O (isocyanate).

De préférence, les groupes B2 sont identiques aux groupes B'2.

De préférence, les composés (A2) sont de structure (C') suivante :

B'2-L'-B'2 (C')

le linker L' ayant les mêmes significations que L défini précédemment.

Dans une version particulièrement préférée de l'invention, les polymères A1 sont de formule (C1) :

CON-L-NCO-X-R X'-CON-L-NCO

(C1)

dans laquelle L, X, X' et R ont les mêmes significations que précédemment.

De préférence, la molécule (A3) est la 6-méthylisocytosine de formule :

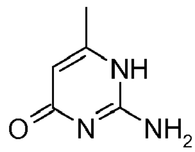

Dans la pratique, le ou les polymères supramoléculaires selon l'invention peuvent être préparés par les procédés usuellement employés par l'homme du métier pour former une liaison uréthane, entre les fonctions OH libres d'un polyalcène polyhydroxylé et les fonctions isocyanates portées par le groupe de jonction. A titre d'illustration, un procédé général de préparation consiste à :

- s'assurer que le polymère à fonctionnaliser ne comporte pas d'eau résiduelle ;
- chauffer le polymère comportant au moins deux fonctions réactives, notamment OH, à une température pouvant être comprise entre 60°C et 140°C. L'indice d'hydroxyle du polymère pourra faire servir de référence afin de mesurer l'état d'avancement de la réaction ;
- ajouter directement le greffon portant les fonctions réactives, notamment isocyanate ;
- agiter le mélange, sous atmosphère contrôlée, à une température de l'ordre de 90-130°C; pendant 1 à 24 heures ;
- suivre par spectroscopie infrarouge, la disparition de la bande caractéristique des isocyanates (compris entre 2500 et 2800 $cm^{-1}$) de manière à arrêter la réaction à la disparition totale du pic, puis à laisser revenir à température

ambiante le produit final ;

- la réaction pourra aussi être suivi par des dosages des fonctions hydroxyles ;
- un ajout d'éthanol pourra être éventuellement effectué afin de s'assurer de la disparition totale de fonctions isocyanate résiduelles ;
- Le mélange pourra être filtré si nécessaire.

La réaction peut être effectuée en présence d'un solvant, notamment la méthyltétrahydrofurane, le tétrahydrofurane, le toluène ou l'acétate de butyle, ou encore le propylène carbonate.

Il est également possible d'ajouter un catalyseur conventionnel de la formation de liaison uréthane. A titre d'exemple, on peut citer le dilaurate dibutyle étain.

Le composé peut au final être lavé et séché, voire purifié, selon les connaissances générales de l'homme du métier.

Selon un autre mode de réalisation, la réaction peut comporter les étapes suivantes :

(i) Fonctionnalisation du polymère polyalcène P polyhydroxylé préalablement séché par un diisocyanate selon le schéma réactionnel suivant :

$$\text{OH - P-OH (1éq) + NCO-X-NCO (1éq)} \rightarrow \text{OCN-X-NH-(O)CO- P-OC(O)-NH-X-NCO}$$

Le diisocyanate peut être éventuellement en excès par rapport au polymère. Cette première étape peut être faite en présence de solvant, à une température comprise entre 20°C et 100°C.

Cette première étape peut être suivie par une période d'agitation, sous atmosphère contrôlée pendant une période allant de 1 heure à 24 heures. Le mélange peut être éventuellement chauffé.

L'état d'avancement de cette première étape peut être suivi par un dosage des fonctions hydroxyles. puis

(ii) réaction du pré-polymère obtenu dans l'étape (i) avec la 6-méthylisocytosine :

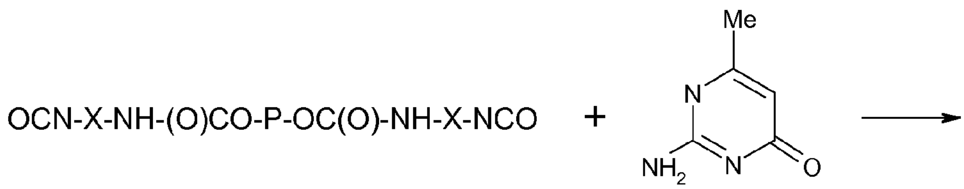

Cette deuxième étape peut éventuellement se faire en présence d'un co-solvant comme le toluène, l'acétate de butyle ou encore le propylène carbonate. Le mélange réactionnel peut être chauffé entre 80°C et 140°C pendant un temps variant entre 1 heure et 24 heures.

La présence d'un catalyseur peut favoriser l'obtention du produit final désiré. Nous pouvons citer par exemple l'utilisation du dilaurate dibutyle étain.

La réaction peut être suivie par spectroscopie infra-rouge, en suivant la disparition du pic caractéristique de l'isocyanate entre 2200 et 2300 $cm^{-1}$.

A la fin de la réaction, de l'éthanol peut être additionné au milieu réactionnel afin de neutraliser les fonctions isocyanates résiduelles. Le mélange réactionnel peut être éventuellement filtré. Pour les besoins d'applications, le polymère pourra être directement strippé dans un solvant cosmétique.

Le ou les polymères supramoléculaires à base de de polyalcène peuvent être présents dans la composition en une teneur allant de 0,1 % à 40 % en poids, de préférence allant de 0,1 % à 30 % en poids, plus préférentiellement allant de 0,5 % à 20 % en poids, et encore plus préférentiellement allant de 1 % à 20 % en poids par rapport au poids total de la composition.

**<u>COPOLYMERE ETHYLENIQUE SEQUENCE</u>**

La composition selon la présente invention contient au moins un copolymère éthylénique séquencé (également appelé polymère éthylénique séquencé), contenant au moins une première séquence ayant une température de transition vitreuse (Tg) supérieure ou égale à 40°C et étant issue en totalité ou en partie de un ou plusieurs premiers monomères, qui sont tels que l'homopolymère préparé à partir de ces monomères a une température de transition vitreuse supérieure

ou égale à 40°C, et au moins une deuxième séquence ayant une température de transition vitreuse inférieure ou égale à 20°C et étant issue en totalité ou en partie de un ou plusieurs deuxièmes monomères, qui sont tels que l'homopolymère préparé à partir de ces monomères a une température de transition vitreuse inférieure ou égale à 20°C, ladite première séquence et ladite deuxième séquence étant reliées entre elles par un segment intermédiaire statistique comprenant au moins un desdits premiers monomères constitutifs de la première séquence et au moins un desdits deuxièmes monomères constitutifs de la deuxième séquence, et ledit copolymère séquencé ayant un indice de polydispersité I supérieur à 2.

Le polymère séquencé utilisé selon l'invention comprend ainsi au moins une première séquence et au moins une deuxième séquence.

Par "au moins" une séquence, on entend une ou plusieurs séquences.

Par polymère "séquencé", on entend un polymère comprenant au moins 2 séquences distinctes, de préférence au moins 3 séquences distinctes.

Par polymère "éthylénique", on entend un polymère obtenu par polymérisation de monomères comprenant une insaturation éthylénique.

Le polymère éthylénique séquencé utilisé selon l'invention est préparé exclusivement à partir de monomères monofonctionnels.

Cela signifie que le polymère éthylénique séquencé utilisé selon la présente invention ne contient pas de monomères multifonctionnels, qui permettent de casser la linéarité d'un polymère afin d'obtenir un polymère branché ou voire réticulé, en fonction du taux de monomère multifonctionnel. Le polymère utilisé selon l'invention ne contient pas non plus de macromonomères (par « macromonomère » on entend un monomère monofonctionnel ayant un groupe pendant de nature polymérique, et ayant de préférence une masse moléculaire supérieure à 500 g/mol, ou bien un polymère comportant sur une seule de ses extrémités un groupe terminal polymérisable (ou à insaturation éthylénique)), qui sont utilisés à la préparation d'un polymère greffé.

On précise que dans ce qui précède et ce qui suit les termes "première" et "deuxième" séquences ne conditionnent nullement l'ordre desdites séquences (ou blocs) dans la structure du polymère.

La première séquence et la deuxième séquence du polymère utilisé dans l'invention peuvent être avantageusement incompatibles l'une avec l'autre.

Par "séquences incompatibles l'une avec l'autre", on entend que le mélange formé par un polymère correspondant à la première séquence et par un polymère correspondant à la deuxième séquence, n'est pas miscible dans le solvant de polymérisation, majoritaire en poids, du polymère séquencé, à température ambiante (25°C) et pression atmosphérique (105 Pa), pour une teneur du mélange desdits polymères supérieure ou égale à 5 % en poids, par rapport au poids total du mélange desdits polymères et dudit solvant de polymérisation, étant entendu que :

i) lesdits polymères sont présents dans le mélange en une teneur telle que le rapport pondéral respectif va de 10/90 à 90/10, et que

ii)chacun des polymères correspondant au première et seconde séquences a une masse moléculaire moyenne (en poids ou en nombre) égale à celle du polymère séquencé +/- 15%.

Dans le cas d'un mélange de solvants de polymérisation, dans l'hypothèse de deux ou plusieurs solvants présents en proportions massiques identiques, ledit mélange de polymères est non miscible dans au moins l'un d'entre eux.

Bien entendu, dans le cas d'une polymérisation réalisée dans un solvant unique, ce dernier est le solvant majoritaire.

Le polymère séquencé selon l'invention comprend au moins une première séquence et au moins une deuxième séquence reliées entre elles par un segment intermédiaire comprenant au moins un monomère constitutif de la première séquence et au moins un monomère constitutif de la deuxième séquence. Le segment intermédiaire (également appelé séquence intermédiaire) a une température de transition vitreuse Tg comprise entre les températures de transition vitreuse des première et deuxième séquences.

Le segment intermédiaire est une séquence comprenant au moins un monomère constitutif de la première séquence et au moins un monomère constitutif de la deuxième séquence du polymère permet de "compatibiliser" ces séquences.

Avantageusement, le segment intermédiaire comprenant au moins un monomère constitutif de la première séquence et au moins un monomère constitutif de la deuxième séquence du polymère est un polymère statistique.

De préférence, la séquence intermédiaire est issue essentiellement de monomères constitutifs de la première séquence et de la deuxième séquence.

Par "essentiellement", on entend au moins à 85%, de préférence au moins à 90%, mieux à 95% et encore mieux à 100%.

Le polymère séquencé selon l'invention est avantageusement un polymère éthylénique séquencé filmogène.

Par polymère "éthylénique", on entend un polymère obtenu par polymérisation de monomères comprenant une insaturation éthylénique.

Par polymère "filmogène", on entend un polymère apte à former à lui seul ou en présence d'un agent auxiliaire de filmification, un dépôt continu sur un support, notamment sur les matières kératiniques.

De façon préférentielle, le polymère selon l'invention ne comprend pas d'atomes de silicium dans son squelette. Par "squelette", on entend la chaîne principale du polymère, par opposition aux chaînes latérales pendantes.

De préférence, le polymère selon l'invention n'est pas hydrosoluble, c'est à dire que le polymère n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1% en poids, à température ambiante (25°C).

De préférence, le polymère selon l'invention n'est pas un élastomère.

Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.

De manière plus spécifique, par "polymère non élastomére" on désigne un polymère ayant une recouvrance instantanée Ri < à 50% et une recouvrance retardée R2h < 70% après avoir subi un allongement de 30%. De préférence, Ri est < à 30 %, et R2h < 50%.

Plus précisément, le caractère non élastomérique du polymère est déterminé selon le protocole suivant :

On prépare un film de polymère par coulage d'une solution du polymère dans une matrice téflonnée puis séchage pendant 7 jours dans une ambiance contrôlée à 23±5°C et 50±10 % d'humidité relative.

On obtient alors un film d'environ 100 μm d'épaisseur dans lequel sont découpées des éprouvettes rectangulaires (par exemple à l'emporte-pièce) d'une largeur de 15 mm et d'une longueur de 80 mm.

On impose à cet échantillon une sollicitation de traction à l'aide d'un appareil commercialisé sous la référence Zwick, dans les mêmes conditions de température et d'humidité que pour le séchage.

Les éprouvettes sont étirées à une vitesse de 50 mm/min et la distance entre les mors est de 50 mm, ce qui correspond à la longueur initiale (l0) de l'éprouvette.

On détermine la recouvrance instantanée Ri de la manière suivante :

- on étire l'éprouvette de 30 % (εmax) c'est-à-dire environ 0,3 fois sa longueur initiale (l0)
- on relâche la contrainte en imposant une vitesse de retour égale à la vitesse de traction, soit 50 mm/min et on mesure l'allongement résiduel de l'éprouvette en pourcentage, après retour à contrainte charge nulle (εi).

La recouvrance instantanée en % (Ri) est donnée par la formule ci-après:

$$Ri = (\varepsilon max - \varepsilon i) / \varepsilon max) \times 100$$

Pour déterminer la recouvrance retardée, on mesure après 2 heures le taux d'allongement résiduel de l'éprouvette en pourcentage (ε2h), 2 heures après retour à la contrainte charge nulle.

La recouvrance retardée en % (R2h) est donnée par la formule ci-après:

$$R2h = (\varepsilon max - \varepsilon 2h) / \varepsilon max) \times 100$$

A titre purement indicatif, un polymère selon un mode de réalisation de l'invention possède de préférence une recouvrance instantanée Ri de 10% et une recouvrance retardée R2h de 30%.

L'indice de polydispersité du polymère de l'invention est supérieur à 2.

Avantageusement, le polymère séquencé utilisé dans les compositions selon l'invention a un indice de polydispersité I supérieur à 2, par exemple allant de 2 à 9, de préférence supérieur ou égal à 2,5, par exemple allant de 2,5 à 8, et mieux supérieur ou égal à 2,8 et notamment, allant de 2,8 à 6.

L'indice de polydispersité I du polymère est égal au rapport de la masse moyenne en poids Mw sur la masse moyenne en nombre Mn.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).

La masse moyenne en poids (Mw) du polymère selon l'invention est de préférence inférieure ou égale à 300 000, elle va par exemple de 35 000 à 200 000, et mieux de 45 000 à 150 000 g/mol.

La masse moyenne en nombre (Mn) du polymère selon l'invention est de préférence inférieure ou égale à 70 000, elle va par exemple de 10 000 à 60 000, et mieux de 12 000 à 50 000 g/mol.

De préférence, l'indice de polydispersité du polymère selon l'invention est supérieur à 2, par exemple allant de 2 à 9, de préférence supérieur ou égal à 2,5, par exemple allant de 2,5 à 8, et mieux supérieur ou égal à 2,8 et notamment, allant de 2,8 à 6.

<u>Première séquence ayant une Tg supérieure ou égale à 40°C</u>

La séquence ayant une Tg supérieure ou égale à 40°C a par exemple une Tg allant de 40 à 150°C, de préférence supérieure ou égale à 50°C, allant par exemple de 50°C à 120°C, et mieux supérieure ou égale à 60°C, allant par exemple de 60°C à 120°C .

Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg = \sum_{i} (\varpi i / Tg\ i)\ ,$$

ω i étant la fraction massique du monomère i dans la séquence considerée et Tgi étant la température de transition vitreuse de l'homopolymère du monomère i.

Sauf indication contraire, les Tg indiquées pour les première et deuxième séquences dans la présente demande sont des Tg théoriques.

L'écart entre les températures de transition vitreuse des première et deuxième séquences est généralement supérieur à 10°C, de préférence supérieur à 20°C, et mieux supérieur à 30°C.

On entend désigner dans la présente invention, par l'expression « compris entre ... et ... », un intervalle de valeurs dont les bornes mentionnées sont exclues, et « de ... à ... » et « allant de ... à ... », un intervalle de valeurs dont les bornes sont inclues.

La séquence ayant une Tg supérieure ou égale à 40°C peut être un homopolymère ou un copolymère.

La séquence ayant une Tg supérieure ou égale à 40°C peut être issue en totalité ou en partie de un ou plusieurs monomères, qui sont tels que l'homopolymère préparé à partir de ces monomères a une température de transition vitreuse supérieure ou égale à 40°C. Cette séquence peut également être appelée « séquence rigide ».

Dans le cas où cette séquence est un homopolymère, elle est issue de monomères, qui sont tel(s) que les homopolymères préparés à partir de ces monomères ont des températures de transition vitreuse supérieures ou égales à 40°C. Cette première séquence peut être un homopolymère, constitué par un seul type de monomère (dont la Tg de l'homopolymère correspondant est supérieure ou égale à 40°C).

Dans le cas où la première séquence est un copolymère, elle peut être issue en totalité ou en partie de un ou de plusieurs monomères, dont la nature et la concentration sont choisies de façon que la Tg du copolymère résultant soit supérieure ou égale à 40°C. Le copolymère peut par exemple comprendre :

- des monomères qui sont tel(s) que les homopolymères préparés à partir de ces monomères ont des Tg supérieures ou égales à 40°C, par exemple une Tg allant de 40°C à 150 °C, de préférence supérieure ou égale à 50°C, allant par exemple de 50°C à 120°C, et mieux supérieure ou égale à 60°C, allant par exemple de 60°C à 120°C, et
- des monomères qui sont tel(s) que les homopolymères préparés à partir de ces monomères ont des Tg inférieures à 40°C, choisis parmi les monomères ayant une Tg comprise entre 20°C à 40°C et/ou les monomères ayant une Tg inférieure ou égale à 20°C, par exemple une Tg allant de -100°C à 20°C, de préférence inférieure à 15°C, notamment allant de - 80°C à 15°C et mieux inférieur à 10°C, par exemple allant de -50°C à 0°C à, tels que décrits plus loin.

Les premiers monomères dont les homopolymères ont une température de transition vitreuse supérieure ou égale à 40°C sont, de préférence, choisis parmi les monomères suivants, appelés aussi monomères principaux :

- les méthacrylates de formule $CH_2 = C(CH_3)\text{-}COOR_1$
  dans laquelle $R_1$ représente un groupe alkyle non substitué, linéaire ou ramifié, contenant de 1 à 4 atomes de

carbone, tel qu'un groupe méthyle, éthyle, propyle ou isobutyle ou $R_1$ représente un groupe cycloalkyle $C_4$ à $C_{12}$, de préférence un cycloalkyle $C_8$ à $C_{12}$, tel que le méthacrylate d'isobornyle,

- les acrylates de formule $CH_2 = CH\text{-}COOR_2$

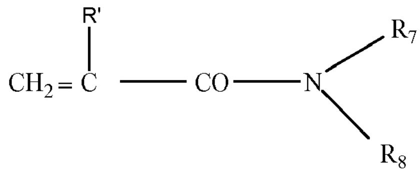

dans laquelle $R_2$ représente un groupe cycloalkyle en $C_4$ à $C_{12}$ tel qu'un groupe isobornyle ou un groupe tertio butyle,

- les (méth)acrylamides de formule :

où $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl,

et R' désigne H ou méthyle. Comme exemple de monomères, on peut citer le N-butylacrylamide, le N-t-butylacrylamide, le N-isopropylacrylamide, le N,N-diméthylacrylamide et le N,N-dibutylacrylamide ,

- et leurs mélanges.

La première séquence est avantageusement obtenue à partir d'au moins un monomère acrylate de formule $CH_2 = CH\text{-}COOR_2$ et d'au moins un monomère méthacrylate de formule $CH_2 = C(CH_3)\text{-}COOR_2$ dans laquelle $R_2$ représente un groupe cycloalkyle $C_4$ à $C_{12}$, de préférence un cycloalkyle $C_8$ à $C_{12}$, tel que l'isobornyle. Les monomères et leurs proportions sont de préférence choisis de telle sorte que la température de transition vitreuse de la première séquence est supérieure ou égale à 40°C.

Selon un mode de mise en oeuvre, la première séquence est obtenue à partir :

i) d'au moins un monomère acrylate de formule $CH_2 = CH\text{-}COOR_2$ dans laquelle $R_2$ représente un groupe cycloalkyle $C_4$ à $C_{12}$, de préférence un groupe cycloalkyle en $C_8$ à $C_{12}$, tel que l'isobornyle,

- ii) et d'au moins un monomère méthacrylate de formule $CH_2 = C(CH_3)\text{-}COOR'_2$ dans laquelle $R'_2$ représente un groupe cycloalkyle $C_4$ à $C_{12}$, de préférence un groupe cycloalkyle en $C_8$ à $C_{12}$, tel que l'isobornyle.

Selon un mode de mise en oeuvre, la première séquence est obtenue à partir d'au moins un monomère acrylate de formule $CH_2 = CH\text{-}COOR_2$ dans laquelle R2 représente un groupe cycloalkyle $C_8$ à $C_{12}$, tel que l'isobornyle, et d'au moins un monomère méthacrylate de formule $CH_2 = C(CH_3)\text{-}COOR'_2$ dans laquelle $R'_2$ représente un groupe cycloalkyle $C_8$ à $C_{12}$, tel que l'isobornyle.

De façon préférée, $R_2$ et $R'_2$ représentent indépendamment ou simultanément un groupe isobornyle.

De façon préférée, le copolymère séquencé comprend de 50 à 80% en poids de méthacrylate/acrylate d'isobornyle, de 10 à 30% en poids d'acrylate d'isobutyle et de 2 à 10% en poids d'acide acrylique.

La première séquence peut être obtenue exclusivement à partir dudit monomère acrylate et dudit monomère méthacrylate.

Le monomère acrylate et le monomère méthacrylate sont de préférence dans des propositions massiques comprises entre 30 :70 et 70 :30, de préférence entre 40 :60 et 60 :40, notamment de l'ordre de 50 :50.

La proportion de la première séquence va avantageusement de 20 à 90% en poids du polymère, mieux de 30 à 80% et encore mieux de 60 à 80%.

Selon un mode de mise en oeuvre, la première séquence est obtenue par polymérisation du méthacrylate d'isobornyle et de l'acrylate d'isobornyle.

<u>Deuxième séquence de température de transition vitreuse inférieure à 20°C.</u>

La deuxième séquence a avantageusement une température de transition vitreuse Tg inférieure ou égale à 20°C a par exemple une Tg allant de -100°C à 20°C, de préférence inférieure ou égale à 15°C, notamment allant de -80°C à 15°C et mieux inférieure ou égale à 10°C, par exemple allant de - 100 °C à 10 °C, notamment allant de -30°C à 10°C.

La deuxième séquence est issue en totalité ou en partie de un ou plusieurs deuxièmes monomères, qui sont tels que l'homopolymère préparé à partir de ces monomères a une température de transition vitreuse inférieure ou égale à 20°C.

Cette séquence peut également être appelée « séquence souple ».

Le monomère ayant une Tg inférieure ou égale à 20°C (appelé deuxième monomère) est, de préférence, choisi parmi les monomères suivants:

- les acrylates de formule $CH_2$ = $CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,
- les méthacrylates de formule $CH_2$ = $C(CH_3)$-$COOR_4$,
  R4 représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule R5-CO-O-CH = CH2
  où R5 représente un groupe alkyle en $C_4$ à $C_{12}$ linéaire ou ramifié ;
- les éthers d'alcool vinylique et d'alcool en $C_4$ à $C_{12}$,
- les N-alkyl en $C_4$ à $C_{12}$ acrylamides, tels que le N-octylacrylamide,
- et leurs mélanges.

Les monomères ayant une Tg inférieure ou égale à 20°C préférés sont l'acrylate d'isobutyle, l'acrylate d'éthyl-2 hexyle ou leurs mélanges en toutes proportions.

Chacune des première et deuxième séquences peut contenir, en proportion minoritaire, au moins un monomère constitutif de l'autre séquence.

Ainsi la première séquence peut contenir au moins un monomère constitutif de la deuxième séquence et inversement.

Chacune des première et/ou deuxième séquence, peu(ven)t comprendre, outre les monomères indiqués ci-dessus, un ou plusieurs autres monomères appelés monomères additionnels, différents des monomères principaux cités précédemment.

La nature et la quantité de ce ou ces monomères additionnels sont choisies de manière à ce que la séquence dans laquelle ils se trouvent ait la température de transition vitreuse désirée.

Ce monomère additionnel est par exemple choisi parmi :

- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
- les méthacrylates de formule CH2 = C(CH3)-COOR6
  dans laquelle R6 représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule CH2 = C(CH3)-COOR9,
  R9 représentant un groupe alkyle en $C_6$ à $C_{12}$ linéaire ou ramifié, dans lequel se trouve(nt) éventuellement intercalé (s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;
- les acrylates de formule $CH_2$ = $CHCOOR_{10}$,

$R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle en $C_1$ à $C_{12}$-O-POE (polyoxyéthylène) avec répétition du motif oxyéthylène de 5 à 10 fois, par exemple méthoxy-POE, ou $R_8$ représente un groupement polyoxyéthylèné comprenant de 5 à 10 motifs d'oxyde d'éthylène.

En particulier, la première séquence peut comprendre à titre de monomère additionnel :

- de l'acide (méth)acrylique, de préférence de l'acide acrylique,
- de l'acrylate de tertiobutyle
- les méthacrylates de formule $CH_2$ = $C(CH_3)$-$COOR_1$
  dans laquelle $R_1$ représente un groupe alkyle non substitué, linéaire ou ramifié, contenant de 1 à 4 atomes de carbone, tel qu'un groupe méthyle, éthyle, propyle ou isobutyle,

- les (méth)acrylamides de formule :

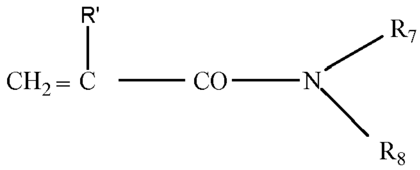

où $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl,

et R' désigne H ou méthyle. Comme exemple de monomères, on peut citer le N-butylacrylamide, le N-t-butylacrylamide, le N-isopropylacrylamide, le N,N-diméthylacrylamide et le N,N-dibutylacrylamide ,

- et leurs mélanges.

Le monomère additionnel peut représenter 0,5 à 30% en poids du poids du polymère. Selon un mode de mise en oeuvre, le polymère de l'invention ne contient pas de monomère additionnel.

De préférence, le polymère de l'invention comprend au moins des monomères acrylate d'isobornyle et méthacrylate d'isobornyle dans la première séquence et des monomères acrylate d'isobutyle et acide acrylique dans la deuxième séquence.

De préférence, le polymère comprend au moins des monomères acrylate d'isobornyle et méthacrylate d'isobornyle en proportion équivalente en poids dans la première séquence et des monomères acrylate d'isobutyle et acide acrylique dans la deuxième séquence.

De préférence, le polymère comprend au moins des monomères acrylate d'isobornyle et méthacrylate d'isobornyle en proportion équivalente en poids dans la première séquence, et des monomères acrylate d'isobutyle et acide acrylique dans la deuxième séquence, la première séquence représentant 70% en poids du polymère.

De préférence, le polymère comprend au moins des monomères acrylate d'isobornyle et méthacrylate d'isobornyle en proportion équivalente en poids dans la première séquence, et des monomères acrylate d'isobutyle et acide acrylique dans la deuxième séquence. De façon préférée, la séquence de Tg supérieure à 40°C représentant 70% en poids du polymère, et l'acide acrylique représentant 5% en poids du polymère.

Selon un mode de réalisation, la première séquence ne comprend pas de monomère additionnel.

Selon un mode de réalisation préféré, la deuxième séquence comprend de l'acide acrylique à titre de monomère additionnel. En particulier, la deuxième séquence est avantageusement obtenue à partir d'un monomère acide acrylique et d'au moins un autre monomère ayant une Tg inférieure ou égale à 20°C.

Le copolymère séquencé peut avantageusement comprendre plus de 2 % en poids de monomères acide acrylique, et notamment de 2 à 15 % en poids, par exemple de 3 à 15 % en poids, en particulier de 4 à 15 % en poids, voire de 4 à 10 % en poids de monomères acide acrylique, par rapport au poids total dudit copolymère.

Les monomères constitutifs de la deuxième séquence et leurs proportions sont choisis de telle sorte que la température de transition vitreuse de la deuxième séquence est inférieure ou égale à 20°C.

Segment intermédiaire

Le segment intermédiaire (également appelé séquence intermédiaire) relie la première séquence et la deuxième séquence du polymère utilisé selon la présente invention. Le segment intermédiaire résulte de la polymérisation :

i) du ou des premiers monomères, et éventuellement du ou des monomères additionnels, restant disponibles après leur polymérisation à un taux de conversion d'au maximum 90% pour former la première séquence,

ii) et du ou des deuxièmes monomères, et éventuellement du ou des monomères additionnels, ajoutés dans le mélange réactionnel.

La formation de la deuxième séquence est initiée lorsque les premiers monomères ne réagissent plus ou ne s'incorporent plus dans la chaine polymérique soit parce qu'ils sont tous consommés soit parce que leur réactivité ne leur permet plus d'être.

Ainsi le segment intermédiaire comprend les premiers monomères disponibles, résultant d'un taux de conversion de ces premiers monomères inférieur ou égal à 90%, lors de l'introduction du ou des deuxièmes monomères lors de la synthèse du polymère.

Le segment intermédiaire du polymère séquencé est un polymère statistique (peut également être appelé une séquence statistique). C'est-à-dire qu'il comprend une répartition statistique du ou des premiers monomères et du ou des deuxièmes monomères ainsi que du ou des monomères additionnels éventuellement présents.

Ainsi, le segment intermédiaire est une séquence statistique, de même que la première séquence et la deuxième séquence si elles ne sont pas des homopolymères (c'est-à-dire si elles sont toutes deux formées à partir d'au moins deux monomères différents).

Procédé de préparation du copolymère :

Le copolymère éthylénique séquencé selon l'invention est préparé par polymérisation radicalaire libre, selon les techniques bien connues de ce type de polymérisation.

La polymérisation radicalaire libre est effectuée en présence d'un amorceur dont la nature est adaptée, de façon connue, en fonction de la température de polymérisation souhaitée et du solvant de polymérisation. En particulier, l'amorceur peut être choisi parmi les amorceurs à fonction peroxyde, les couples d'oxydoréduction, ou d'autres amorceurs de polymérisation radicalaire connus de l'homme de l'art.

En particulier, à titre d'amorceur à fonction peroxyde, on peut citer par exemple:

a. les péroxyesters, tel que le terbutyl-péroxyacétate, le perbenzoate de tertiobutyle, le tertbutyl péroxy-2-éthylhexanoate (Trigonox 21 S d'Akzo Nobel), le 2,5-bis(2-éthylhexanoylpéroxy)-2,5-diméthylhexane (Trigonox 141 d'Akzo Nobel) ;
b. les péroxydicarbonates, tel que le di-isopropylpéroxydicarbonate ;
c. les péroxycetones, tel que le méthyléthylcétone péroxyde ;
d. hydropéroxydes, tel que l'eau oxygénée (H2O2), le terbutylhydropéroxyde ;
e. les péroxydes de diacyle, tel que l'acétyl péroxyde, le benzoyl péroxyde ;
f. les péroxydes de dialkyle, tel que le di-tertiobutyle péroxyde ;
g. les péroxydes inorganiques, tel que le péroxodisulfate de potassium (K2S2O8);

A titre d'amorceur sous forme de couple d'oxydoréduction, on peut citer le couple thiosulfate de potassium + peroxodisulfate de potassium par exemple.

Selon un mode de réalisation préférée, l'amorceur est choisi parmi les peroxydes organiques comprenant de 8 à 30 atomes de carbone. De façon préférée, l'amorceur utilisé est le 2.5- Bis(2-éthylhexanoylperoxy)-2.5-diméthylhexane commercialisé sous la référence Trigonox® 141 par la société Akzo Nobel.

Le copolymère séquencé utilisé selon l'invention est préparé par polymérisation radicalaire libre et non par polymérisation contrôlée ou vivante. En particulier, la polymérisation du copolymère éthylénique séquencé est réalisée en l'absence d'agents de contrôle, et en particulier en l'absence d'agent de contrôle classiquement utilisés dans les procédés de polymérisation vivante ou contrôlée tels que les nitroxydes, les alcoxyamines, les dithioesters, les dithiocarbamates, les dithiocarbonates ou xanthates, les trithiocarbonates, les catalyseurs à base de cuivre, par exemple.

Comme indiqué précédemment, le segment intermédiaire est une séquence statistique, de même que la première séquence et la deuxième séquence si elles ne sont pas des homopolymères (c'est-à-dire si elles sont toutes deux formées à partir d'au moins deux monomères différents).

Le copolymère séquencé peut être préparé par polymérisation radicalaire libre, et en particulier par un procédé consistant à mélanger, dans un même réacteur, un solvant de polymérisation, un amorceur, au moins un monomère de transition vitreuse supérieure ou égale à 40°C, au moins un monomère de transition vitreuse inférieure ou égale à 20 °C selon la séquence suivante :

- on verse dans le réacteur, une partie du solvant de polymérisation et éventuellement une partie de l'amorceur et des monomères de la première coulée, mélange que l'on chauffe à une température de réaction comprise entre 60 et 120 °C,
- on verse ensuite, en une première coulée, ledit au moins un premier monomère de Tg supérieure ou égale à 40°C et éventuellement une partie de l'amorceur que l'on laisse à réagir pendant une durée T correspondant à un taux de conversion desdits monomères de 90 % maximum,
- on verse ensuite dans le réacteur, en une deuxième coulée, à nouveau de l'amorceur de polymérisation, ledit au moins un deuxième monomère de transition vitreuse inférieure ou égale à 20 °C, qu'on laisse réagir pendant une durée T' au bout de laquelle le taux de conversion desdits monomères atteint un plateau,
- on ramène le mélange réactionnel à température ambiante.

De façon préférée, le copolymère peut être préparé par polymérisation radicalaire libre, en particulier par un procédé consistant à mélanger, dans un même réacteur, un solvant de polymérisation, un amorceur, un monomère

acide acrylique, au moins un monomère de transition vitreuse inférieure ou égale à 20 °C, au moins un monomère acrylate de formule $CH_2$ = CH-$COOR_2$ dans laquelle $R_2$ représente un groupe cycloalkyle $C_4$ à $C_{12}$, et au moins un monomère méthacrylate de formule $CH_2$ = C($CH_3$)-COOR'$_2$ dans laquelle R'$_2$ représente un groupe cycloalkyle $C_4$ à $C_{12}$, selon la séquence d'étape suivante :

- on verse dans le réacteur, une partie du solvant de polymérisation et éventuellement une partie de l'amorceur et des monomères de la première coulée, mélange que l'on chauffe à une température de réaction comprise entre 60 et 120 °C,
- on verse ensuite, en une première coulée, ledit au moins monomère acrylate de formule $CH_2$ = CH-$COOR_2$ et ledit au moins monomère méthacrylate de formule $CH_2$ = C($CH_3$)-COOR'$_2$ en tant que monomères de Tg supérieure ou égale à 40°C, et éventuellement une partie de l'amorceur que l'on laisse à réagir pendant une durée T correspondant à un taux de conversion desdits monomères de 90 % maximum,
- on verse ensuite dans le réacteur, en une deuxième coulée, à nouveau de l'amorceur de polymérisation, le monomère acide acrylique et ledit au moins monomère de transition vitreuse inférieure ou égale à 20 °C, qu'on laisse réagir pendant une durée T' au bout de laquelle le taux de conversion desdits monomères atteint un plateau,
- on ramène le mélange réactionnel à température ambiante.

Par solvant de polymérisation, on entend un solvant ou un mélange de solvants. En particulier, à titre de solvant de polymérisation utilisable on peut citer :

- les cétones liquides à température ambiante tels que méthyléthylcétone, méthylisobutylcétone, diisobutylcétone, l'isophorone, la cyclohexanone, l'acétone ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, l'isododécane, le cyclohexane, l'isohexadécane ;
- les composés cycliques aromatiques liquides à température ambiante tels que le toluène et le xylène ; les aldéhydes liquides à température ambiante tels que le benzaldéhyde, l'acétaldéhyde et leurs mélanges.

Classiquement, le solvant de polymérisation est une huile volatile de point éclair inférieur à 80°C. Le point éclair est mesuré en particulier selon la Norme Iso 3679.
Le solvant de polymérisation peut être choisi notamment parmi l'acétate d'éthyle, l'acétate de butyle, les alcools tels que l'isopropanol, l'éthanol, les alcanes aliphatiques tels que l'isododécane et leurs mélanges. De préférence, le solvant de polymérisation est un mélange acétate de butyle et isopropanol ou l'isododécane.
Selon un autre mode de mise en oeuvre, le copolymère peut être préparé par polymérisation radicalaire libre selon un procédé de préparation, consistant à mélanger, dans un même réacteur, un solvant de polymérisation, un amorceur, au moins un monomère de transition vitreuse inférieure ou égale à 20 °C, et au moins un monomère de Tg supérieure ou égale à 40°C, selon la séquence d'étape suivante :

- on verse dans le réacteur, une partie du solvant de polymérisation et éventuellement une partie de l'amorceur et des monomères de la première coulée, mélange que l'on chauffe à une température de réaction comprise entre 60 et 120 °C,
- on verse ensuite, en une première coulée, ledit au moins un monomère de transition vitreuse inférieure ou égale à 20 °C et éventuellement une partie de l'amorceur que l'on laisse à réagir pendant une durée T correspondant à un taux de conversion desdits monomères de 90 % maximum,
- on verse ensuite dans le réacteur, en une deuxième coulée, à nouveau de l'amorceur de polymérisation, ledit au moins un monomère de Tg supérieure ou égale à 40°C, qu'on laisse réagir pendant une durée T' au bout de laquelle le taux de conversion desdits monomères atteint un plateau,
- on ramène le mélange réactionnel à température ambiante.

Selon un mode préféré de mise en oeuvre, le copolymère peut être préparé par polymérisation radicalaire libre selon un procédé de préparation, consistant à mélanger, dans un même réacteur, un solvant de polymérisation, un amorceur, un monomère acide acrylique, au moins un monomère de transition vitreuse inférieure ou égale à 20°C, au moins un monomère de Tg supérieure ou égale à 40°C, et en particulier en tant que monomères de Tg supérieure ou égale à 40°C, au moins un monomère acrylate de formule $CH_2$ = CH-$COOR_2$ dans laquelle $R_2$ représente un groupe

cycloalkyle $C_4$ à $C_{12}$, et au moins un monomère méthacrylate de formule $CH_2 = C(CH_3)$-COOR'$_2$ dans laquelle R'$_2$ représente un groupe cycloalkyle $C_4$ à $C_{12}$, selon la séquence d'étape suivante :

- on verse dans le réacteur, une partie du solvant de polymérisation et éventuellement une partie de l'amorceur et des monomères de la première coulée, mélange que l'on chauffe à une température de réaction comprise entre 60 et 120 °C,
- on verse ensuite, en une première coulée, le monomère acide acrylique et ledit au moins monomère de transition vitreuse inférieure ou égale à 20 °C et éventuellement une partie de l'amorceur que l'on laisse à réagir pendant une durée T correspondant à un taux de conversion desdits monomères de 90 % maximum,
- on verse ensuite dans le réacteur, en une deuxième coulée, à nouveau de l'amorceur de polymérisation, ledit au moins un monomère acrylate de formule $CH_2$ = CH-COOR$_2$ et ledit au moins un monomère méthacrylate de formule $CH_2 = C(CH_3)$-COOR'$_2$, en tant que monomère de Tg supérieure ou égale à 40°C, qu'on laisse réagir pendant une durée T' au bout de laquelle le taux de conversion desdits monomères atteint un plateau,
- on ramène le mélange réactionnel à température ambiante.

La température de polymérisation est de préférence de l'ordre de 90 °C.
La durée de réaction après la deuxième coulée est de préférence comprise entre 3 et 6 heures.

Distillation du solvant de synthèse

Pour la mise en oeuvre du polymère séquencé dans une composition selon l'invention, et lorsque le polymère est préparé dans un solvant volatil ou une huile volatile ayant un point éclair inférieur à 80°C, il est nécessaire de procéder à une étape d'élimination totale ou partielle dudit solvant ou huile volatile. On procède en particulier par distillation, éventuellement sous vide, et ajout d'huile ester hydrocarbonée non volatile comprenant au moins 16 atomes de carbone et ayant une masse molaire inférieure à 650 g/moles.
Cette technique est connue de l'homme du métier. La distillation du solvant de synthèse (classiquement l'isododécane) est réalisée avec ajout simultané ou en présence dans le mélange avant la distillation d'une huile ester hydrocarbonée non volatile comprenant au moins 16 atomes de carbone et ayant une masse molaire inférieure à 650 g/mole. Cette étape est réalisée à chaud et éventuellement sous vide pour distiller un maximum d'isododécane (et plus généralement de solvant de synthèse), si celui-ci a été utilisé en tant que solvant de polymérisation, ou plus généralement pour distiller un maximum d'huile volatile dont le point éclair est inférieur à 80°C. L'huile ester non volatile peut également être ajoutée en partie ou intégralement au polymère dans le solvant volatil avant la distillation.
L'élimination de l'huile volatile de point éclair inférieur à 80°C (classiquement l'isododécane), permet de limiter la teneur de celle-ci dans la solution de copolymère séquencé et ainsi de réaliser une composition cosmétique contenant moins de 10% en poids d'isododécane (et plus généralement de solvant volatil) et de préférence moins de 5 % en poids d'isododécane (et plus généralement de solvant volatil), par rapport au poids total de la composition.
La composition selon l'invention comprend de préférence moins de 0,5 à 40 % en poids de copolymère éthylénique séquencé, et avantageusement de 1 à 40 % en poids, notamment de 2 à 30 % en poids, voire de 2 à 20 % en poids de matière active par rapport au poids total de la composition.

**Solvant volatil**

Selon l'invention, la composition contient un ou plusieurs solvants volatils.
Dans le cadre de l'invention, on entend par solvant volatil un composé liquide à la température ambiante (20 °C) et à la pression atmosphérique (760 mmHg) présentant une pression de vapeur à 20 °C supérieure à 0,1 mmHg et de préférence comprise entre 0,1 et 300 mmHg, encore plus préférentiellement entre 0,5 et 200 mmHg.
Ce solvant volatil peut être l'eau, un solvant organique non siliconé, un solvant organique siliconé ou leurs mélanges.
A titre de solvant organique non siliconé volatil, on peut citer :

- les alcanols volatils en $C_1$-$C_4$ tels que l'éthanol, l'isopropanol ;
- les alcanes volatils en $C_5$-$C_7$ tels que le n-pentane, l'hexane, le cyclopentane, le 2,3-diméthylbutane, le 2,2-diméthylbutane, le 2-méthylpentane, le 3-méthylpentane ;
- les esters d'acides en $C_1$-$C_{20}$ liquides et d'alcools en $C_1$-$C_8$ volatils tels que l'acétate de méthyle, l'acétate de n-butyle, l'acétate d'éthyle, l'acétate de propyle, l'acétate d'isopentyle, le 3-éthoxypropionate d'éthyle ;
- les cétones liquides à température ambiante et volatiles telles que la méthyléthylcétone, la méthylisobutylcétone, la diisobutylcétone, l'isophorone, la cyclohexanone, l'acétone ;
- les polyols volatils tels que le propylène glycol ;

- les éthers volatils tels que le diméthoxyméthane, le diéthoxyéthane, le diéthyléther ;
- les éthers de glycol volatils comme le 2-butoxyéthanol, le butyle diglycol, le monométhyléther de diéthylène glycol, le n-butyléther de propylène glycol, l'acétate de monométhyl éther de propylène glycol ;
- les huiles hydrocarbonées volatiles telles que les huiles volatiles hydrocarbonées ayant de 8 à 16 atomes de carbone et leurs mélanges, et notamment les alcanes ramifiés en $C_8$-$C_{16}$ comme les iso-alcanes (appelées aussi isoparaffines) en $C_8$-$C_{16}$, l'isododécane, l'isodécane, et par exemple les huiles vendues sous les noms commerciaux d'Isopars ou de Permetyls, et leurs mélanges. On peut aussi citer les néopentanoate d'isohexyle ou d'isodecyle ;

L'huile hydrocarbonée volatile peut également être un alcane volatil linéaire choisi parmi les alcanes linéaires volatils comprenant de 8 à 17 atomes de carbone, et en particulier de 9 à 15 atomes de carbone, et plus particulièrement de 11 à 13 atomes de carbone, notamment d'origine végétale.

A titre d'exemple d'alcane linéaire volatil convenant à l'invention, on peut citer le n-nonadécane ($C_9$), le n-décane ($C_{10}$), le n-undécane ($C_{11}$), le n-dodécane ($C_{12}$), le n-tridécane ($C_{13}$), le n-tétradécane ($C_{14}$), le n-pentadécane ($C_{15}$), le n-héxadécane ($C_{16}$).

- les perfluoroalcanes volatils en $C_4$-$C_{10}$ tels que le dodécafluoropentane, le tétradécafluorohexane, le décafluoropentane ;
- les perfluorocycloalkyles volatils tels que le perfluorométhylcyclopentane, le 1,3-perfluorodiméthylcyclohexane et le perfluorodecaline, vendus respectivement sous les dénominations de "Flutec PC1®", "Flutec PC3®" et "Flutec PC6®" par la Société F2 Chemicals, ainsi que le perfluorodiméthylcyclobutane et la perfluoromorpholine ;
- les composés fluoroalkyles ou hétérofluoroalkyles volatils répondant à la formule suivante :

$$CH_3\text{-}(CH_2)_n\text{-}[Z]t\text{-}X\text{-}CF_3$$

dans laquelle t est 0 ou 1 ; n est 0, 1, 2 ou 3 ; X est un radical perfluoroalkyle divalent, linéaire ou ramifié, ayant de 2 à 5 atomes de carbone, et Z représente O, S, ou NR, R étant un atome d'hydrogène, un radical $-(CH_2)_n-CH_3$ ou un radical $-(CF_2)m-CF_3$, m étant 2, 3, 4 ou 5.

Parmi les composés fluoroalkyles ou hétérofluoroalkyles volatils, on peut notamment citer le méthoxynonafluorobutane vendu sous la dénomination de "MSX 4518®", "HFE-7100®" par la Société 3M et l'éthoxynonafluorobutane vendu sous la dénomination de "HFE-7200®" par la Société 3M.
De préférence, le solvant est choisi de telle manière que son point d'ébullition soit inférieur à 200 °C.

Selon un mode de réalisation particulier, le solvant organique non siliconé est choisi parmi l'éthanol, l'isopropanol, l'acétone, les alcanes liquides à 25 °C et à pression atmosphérique (760 mmHg) tels que l'isododécane.

A titre de solvant siliconé volatil, on peut citer les composés siliconés à faible viscosité choisi parmi les silicones linéaires ou cycliques ayant de 2 à 7 atomes de silicium, ces silicones comportant éventuellement des groupes alkyle ou alkoxy ayant de 1 à 10 atomes de carbone, par exemple l'octaméthylcyclotétrasiloxane, la décaméthylcyclopentasiloxane, la dodécaméthylcyclohexasiloxane, l'heptaméthylhexyltri-siloxane, l'heptaméthyléthyltrisiloxane, l'heptaméthyloctyltrisiloxane, l'octaméthyltrisiloxane, la décaméthyltétrasiloxane, et leurs mélanges. Selon un mode de réalisation particulier, le composé siliconé est choisi parmi la cyclopentadiméthylsiloxane, la dodécaméthylcyclohexasiloxane l'octaméthyltrisiloxane et la décaméthyltétrasiloxane.
Selon un mode de réalisation particulier, le solvant siliconé volatil présente une viscosité inférieure à 50 centistokes.

De préférence, la silicone volatile est choisie parmi la décaméthylcyclopentasiloxane, la dodécaméthylcyclohexasiloxane, l'octaméthyltrisiloxane et la décaméthyltétrasiloxane.

A titre d'exemple, on peut citer la décaméthylcyclopentasiloxane commercialisée sous le nom DC-245 par la société Dow Corning, la dodécaméthylcyclohexasiloxane commercialisée sous le nom DC-246 par la société Dow Corning, l'octaméthyltrisiloxane commercialisée sous le nom DC-200 Fluid 1 cst par la société Dow Corning et la décaméthyltétrasiloxane commercialisée sous le nom DC-200 Fluid 1,5 cst par la société Dow Corning.

Selon un mode de réalisation particulier de l'invention, le ou les solvants volatils sont choisis parmi l'eau, l'éthanol, l'isopropanol, l'acétone, les alcanes volatils tels que définis précédemment et en particulier l'isododécane, le décaméthylcyclopentasiloxane, l'octaméthyltrisiloxane et le décaméthyltétrasiloxane et leurs mélanges.

Le solvant volatil peut être présent dans la composition utile dans le procédé de l'invention en une teneur allant de 0,1% à 95% en poids par rapport au poids total de la composition, de préférence allant de 1% à 90% en poids, et préférentiellement allant de 5% à 90% en poids.

## Composés siliconés additionnels

Afin d'obtenir un meilleur étalement de la composition de l'invention ainsi qu'un gainage amélioré, la composition

de l'invention peut aussi contenir un ou plusieurs polysiloxanes présentant une viscosité supérieure à 100 cst, préférentiellement supérieure à 300 cst. La viscosité de ces polysiloxanes peut être mesurée selon la norme ASTM D-445. De tels polysiloxanes peuvent être des huiles, des gommes ou des résines de silicone, les silicones réticulées.

A titre de polysiloxanes de viscosité supérieure à 100 cst, on peut notamment citer les polydiméthylsiloxanes; les alkyldiméthicones; les polyphénylméthylsiloxanes tels que les phényldiméthicones, les phényltriméthicones, et les vinylméthylméthicones; ainsi que les silicones modifiées par des groupements aliphatiques et/ou aromatiques, éventuellement fluorés, ou par des groupements fonctionnels tels que des groupements hydroxyles, thiols et/ou amines.

De tels polysiloxanes peuvent choisis parmi les silicones de formule (I) :

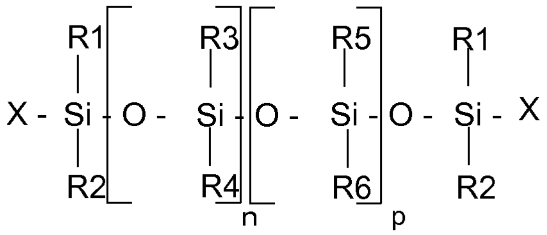

(I)

dans laquelle :

$R_1$, $R_2$, $R_5$ et $R_6$ sont, ensemble ou séparément, un radical alkyle ayant 1 à 6 atomes de carbone, $R_3$ et $R_4$ sont, ensemble ou séparément, un radical alkyle ayant de 1 à 6 atomes de carbone, un radical vinyle, un radical aryle, un radical aminoalkyle ayant de 1 à 6 atomes de carbone éventuellement substitué, un radical hydroxyle, un radical thioalkyle ayant de 1 à 6 atomes de carbone, X est un radical alkyle ayant de 1 à 6 atomes de carbone, un radical hydroxyle, un radical vinyle, un radical aminoalkyle ayant de 1 à 6 atomes de carbone éventuellement substitué, un radical thioalkyle ayant de 1 à 6 atomes de carbone, n et p étant des entiers choisis de manière à obtenir une viscosité supérieure à 300 cst.

A titre d'exemple, on peut citer les polydiméthylsiloxanes suivantes :

- les substituants $R_1$ à $R_6$ et X représentent un groupement méthyle, comme celle vendue sous la dénomination Baysilicone TP 3898 par la société Général Electric, et celle vendue sous la dénomination AK 500000 par la société Waker,
- les substituants $R_1$ à $R_6$ et X représentent un groupement méthyle, p et n sont tels que le poids moléculaire est de 120 000 g/mol, comme celle vendue sous la dénomination Dow Corning 200 Fluid 60000 CS par la société Dow Corning,
- les substituants $R_1$ à $R_6$ et X représentent un groupement méthyle, p et n sont tels que le poids moléculaire est de 250 000 g/mol, comme celle vendue sous la dénomination Mirasil DM 500.000 par la société Rhodia et celle vendue sous la dénomination Dow Corning 200 Fluid 500.000 cst par la société Dow Corning,
- les substituants $R_1$ à $R_6$ représentent un groupement méthyle, le groupement X représente un groupement hydroxy, n et p sont tels que le poids moléculaire du polymère soit de 600 000 g/mol, comme celle vendue sous la dénomination SGM 36 par la société Dow Corning,
- les diméthicones du type (polydiméthylsiloxane)(méthylvinylsiloxane) telle que la SE63 commercialisée par GE BAYER Silicones, les copolymères poly(diméthylsiloxane)(diphényl)(méthylvinylsiloxane), et leurs mélanges.

Dans le cas où le polysiloxane comprend un groupement fluoré, on peut choisir les copolymères de structure suivante :

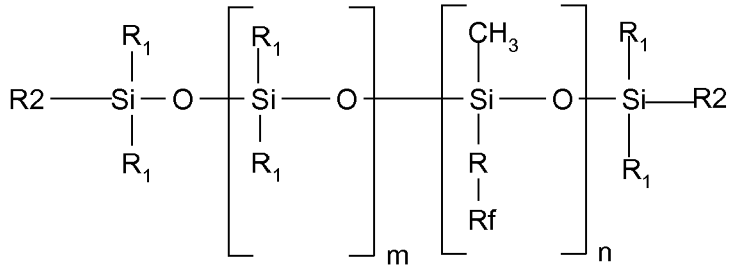

dans laquelle :

R représente un groupement divalent alkyle linéaire ou ramifié, ayant 1 à 6 atomes de carbone, de préférence un groupement divalent méthyle, éthyle, propyle ou butyle, Rf représente un radical fluoroalkyle, notamment un radical perfluoroalkyle, ayant 1 à 12 atomes de carbone, de préférence 1 à 9 atomes de carbone, $R_1$ représente, indépendamment l'un de l'autre, un radical alkyle en $C_1$-$C_{20}$, un radical hydroxyle, un radical phényle, $R_2$ représente $R_1$ ou $R_f$, m est choisi de 0 à 500, de préférence de 0 à 200, et n est choisi de 1 à 1000, de préférence de 1 à 500.

De préférence, les groupements R1 sont identiques et représentent un radical méthyle.

De tels polysiloxanes sont notamment ceux commercialisés par la société Shin Etsu sous les dénominations 'FL-5', 'FL-10', 'X22-821' et 'X22-822' ou encore 'FL-100', par la société Dow Corning sous le nom FS-1265 Fluid, par la société Phoenix Chemical sous la gamme Pecosil FS sous les dénominations Pecosil FSL-150, Pecosil FSL-300, Pecosil FSH-150, Pecosil FSH-300, Pecosil FSU-150, Pecosil FSU-300.

La masse moléculaire en poids du ou des polysiloxane peut être comprise entre 1000 et 1 500 000 g/mol, notamment entre 20 000 et 1 000 000 g/mol.

Le polysiloxane peut être une résine. Par le terme « résine », on entend une structure tridimensionnelle réticulée ou non. A titre d'exemple de résine de polysiloxane, on peut citer les silsesquioxanes et les siloxysilicates.

La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

La lettre M représente l'unité monofonctionelle de formule $(CH_3)_3SiO_{1/2}$, l'atome de silicium étant relié à un seul atome d'oxygène dans le polymère comprenant cette unité.

La lettre D signifie une unité difonctionnelle $(CH_3)_2SiO_{2/2}$ dans laquelle l'atome de silicium est relié à deux atomes d'oxygène

La lettre T représente une unité trifonctionnelle de formule $(CH_3)SiO_{3/2}$.

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.

Enfin, la lettre Q signifie une unité tetrafonctionnelle $SiO_{4/2}$ dans laquelle l'atome de silicium est lié à quatre atomes d'hydrogène eux mêmes liés au reste du polymère.

Divers résines de propriétés différentes peuvent être obtenues à partir de ces différentes unités, les propriétés des ces polymères variant en fonction du type de monomères (ou unités), du type et du nombre de radicaux subsitués, de la longueur de la chaîne polymérique, du degré de ramification et de la taille des chaines pendantes.

A titre d'exemple de ces résines silicones, on peut citer :

- les siloxysilicates qui peuvent être des triméthylsiloxysilicate de formule $[(CH_3)_3SiO]_x(SiO_{4/2})_y$ (unités MQ) dans laquelle x et y sont des entiers allant de 50 à 80,
- les polysilesquioxanes de formule $(CH_3SiO_{3/2})_x$ (unités T) dans laquelle au moins un des radicaux méthyle peut être substitué par un groupement R tel que défini plus haut. De préférence le nombre x d'unités T du silsesquioxane est inférieur ou égal à 500, il est plus préférentiellement compris entre 50 et 500. Le poids moléculaire de la résine de silicone selon l'invention est donc de préférence compris entre 500 et 50 000 g/mol, plus préférentiellement compris entre 500 et 20 000 g/mol et encore plus préférentiellement compris entre 500 et 10 000 g/mol ;
- les polyméthylsilsesquioxanes qui sont des polysilsesquioxanes dans lesquels aucun des radicaux méthyle n'est substitué par un autre groupement. De tels polymethylsilsesquioxanes sont décrits dans le document US 5 246 694 dont le contenu est incorporé par référence ;
- les polypropylsilsesquioxanes, pour lesquelles les radicaux méthyle sont remplacés par des radicaux propyle. Ces

composés ainsi que leur synthèse sont notamment décrit dans la demande de brevet WO 2005/075567 ;

- les polyphénylsilsesquioxanes, pour lesquelles les radicaux méthyle sont remplacés par des radicaux phényle. Ces composés ainsi que leur synthèse sont notamment décrit dans la demande de brevet US 2004/0180011.

A titre d'exemples de résines polyméthylsilsesquioxanes commercialement disponibles, on peut citer celles qui sont commercialisées :

- par la société Wacker sous la référence Resin MK tels que la Belsil PMS MK : polymère comprenant des unités répétitives $CH_3SiO_{3/2}$ (unités T), pouvant aussi comprendre jusqu'à 1% en poids d'unités $(CH_3)_2SiO_{2/2}$ (unités D) et présentant un poids moléculaire moyen d'environ 10 000 g/mol. On pense que le polymère se trouve dans une configuration « cage » et « échelle » comme cela est représenté dans les figures ci-dessous. Le poids moléculaire moyen des unités en configuration « cage » a été calculé à 536 g/mol. La majorité du polymère se trouve en configuration « échelle » avec des groupes éthoxy aux extrémités. Ces groupes éthoxy représentent 4,5 % en masse du polymère. Comme ces extrémités peuvent réagir avec l'eau, un taux faible et variable de groupes SiOH peut également être présent.

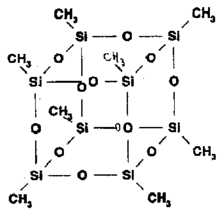

Cage

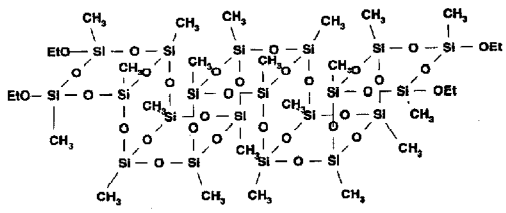

Echelle

- par la société SHIN-ETSU sous les références KR-220L qui sont composées d'unités T de formule $CH_3SiO_{3/2}$ et ont des groupes terminaux Si-OH (silanol), sous la référence KR-242A qui comprennent 98 % d'unités T et 2 % d'unités diméthyle D et ont des groupes terminaux Si-OH ou encore sous la référence KR-251 comprenant 88 % d'unités T et 12 % d'unités diméthyle D et ont des groupes terminaux Si-OH.

A titre d'exemples de résines polypropylsilsesquioxanes commercialement disponibles, on peut citer celles qui sont commercialisées :

- par la société DOW CORNING sous la référence Dow Corning 670 Fluid qui est une polypropylsilsesquioxane diluée dans la D5.

A titre d'exemples de résines polyphénylsilsesquioxanes commercialement disponibles, on peut citer celles qui sont commercialisées :

- par la société DOW CORNING sous la référence Dow Corning 217 Flake Resin qui est une polyphénylsilsesquioxane aux extrémités silanol ;
- par la société WACKER sous la référence Belsil SPR 45 VP.

Comme résines siloxysilicates, on peut citer les résines triméthylsiloxysilicate (TMS) éventuellement sous forme de poudres. De telles résines sont commercialisées sous la référence SR1000 par la société General Electric ou sous la référence TMS 803 par la société Wacker. On peut encore citer les résines triméthylsiloxysilicate commercialisées dans un solvant tel que la cyclométhicone, vendues sous la dénomination "KF-7312J" par la société Shin-Etsu, "DC 749", "DC 593" par la société Dow Corning.

La résine de silicone selon l'invention est de préférence filmogène. En effet, tous les silsesquioxanes ne sont pas filmogènes, par exemple les polyméthylsilsesquioxanes hautement polymérisées comme les TospearlTM de Toshiba ou la KMP590 de Shin-Etsu sont insolubles et ne sont pas filmogènes.

Dans un mode de réalisation de l'invention, la ou les résines de silicones sont solubles ou dispersables dans la composition de l'invention. De préférence, les résines de silicone selon l'invention sont solubles dans les silicones volatiles et les solvants organiques. Dans un mode de réalisation, la résine de silicone est solide à 25 °C.

Les résines de silicones préférées selon l'invention sont les résines triméthylsiloxysilicates, les résines polyméthylsilsesquioxanes et les résines polypropylsilsesquioxanes.

La composition de l'invention peut aussi contenir une silicone réticulée telle qu'un organopolysiloxane élastomère réticulé, un composé siliconé de haut poids moléculaire présentant une structure tridimensionnelle, aux propriétés viscoélastiques d'un matériau solide souple. Ces organopolysiloxanes peuvent ainsi se présenter sous forme sèche en poudre, ou sous forme gonflée, dans un solvant, le produit résultant étant généralement un gel. Ces produits peuvent également se présenter sous forme dispersée dans une solution aqueuse.
La synthèse de ces organopolysiloxane est décrite dans les brevets suivants :

- US 5 266 321 de Kobayashi Kose,
- US 4 742 142 de Toray Silicone,
- US 5 654 362 de Dow Corning Corp,
- la demande de brevet FR 2 864 784.

Les organopolysiloxanes élastomères utilisés dans la composition peuvent être partiellement ou totalement réticulés. Ils se présentent généralement sous forme de particules. En particulier, les particules d'organopolysiloxane élastomère ont une taille moyenne en nombre allant de 0,1 à 500 $\mu$m,. Ces particules peuvent avoir toute forme et par exemple être sphériques, plates ou amorphes.
L'organopolysiloxane réticulé obtenu peut être un composé non-émulsionnant ou un composé émulsionnant. Le terme "non émulsionnant" défini des organopolysiloxanes réticulés ne contenant pas de motifs polyoxyalkylène. Le terme "émulsionnant" signifie des composés organopoysiloxanes réticulés ayant au moins un motif polyoxyalkylène, notamment polyoxyéthylène ou polyoxypropylène.
Les particules d'organopolysiloxane réticulé peuvent être véhiculées sous forme de gel constitué d'un organopolysiloxane réticulé inclus dans au moins une huile hydrocarbonée et/ou une huile siliconée. Dans ces gels, les particules d'organopolysiloxane sont souvent des particules non-sphériques. Les particules d'organopolysiloxane réticulé peuvent également se présenter sous forme de poudre, notamment sous forme de poudre sphérique.
Des organopolysiloxanes réticulés non-émulsionnants sont notamment décrits dans les brevets US 4 970 252, US 4 987 169, US 5 412 004, US 5 654 362, US 5 760 116, dans la demande JP-A-61-194009.
Comme organopolysiloxanes réticulés non-émulsionnants, on peut utiliser ceux vendus sous les dénominations "KSG-6", "KSG-15", "KSG-16", "KSG-18", "KSG-31", "KSG-32", "KSG-33", "KSG-41 ", "KSG-42", "KSG-43", "KSG-44" « USG-103 » par la société Shin Etsu, "DC 9040", "DC9041", "DC 9509", "DC9505", "DC 9506" « DC 9045 » par la société Dow Corning, "GRANSIL" par la société Grant Industries, "SFE 839" par la société General Electric.
Avantageusement, les organopolysiloxanes réticulés émulsionnants comprennent les organopolysiloxanes modifiés polyoxyalkylène formé à partir de composés divinyliques, en particulier des polysiloxanes ayant au moins deux groupes vinyliques, réagissant avec des liaisons Si-H d'un polysiloxane. Des organopolysiloxanes réticulés émulsionnants sont notamment décrits dans les brevets US 5 236 986, US 5 412 004, US 5 837 793, US 5 811 487.
Comme organopolysiloxanes réticulés émulsionnants, on peut utiliser ceux commercialisés sous les dénominations "KSG-21 ", "KSG-20", "KSG-30"par la société Shin Etsu, et "DC901 0", "DC9011" par la société Dow Corning.
Les particules d'organopolysiloxane réticulé élastomère peuvent se présenter également sous forme de poudre d'organopolysiloxane réticulé élastomère enrobée de résine de silicone, notamment de résine silsesquioxane, comme décrit par exemple dans le brevet US 5 538 793.
De tels élastomères sont vendus sous les dénomination "KSP-100", "KSP-1 01 ", "KSP-102", "KSP-103", KSP-104", "KSP-105" par la société Shin Etsu.
De préférence l'organopolysiloxane réticulé est non-émulsionnant.
La composition de l'invention peut aussi contenir un polymère siliconé greffé. Dans le cadre de l'invention, on entend par polymère siliconé greffé, un polymère comprenant une portion polysiloxane et une portion constituée d'une chaîne organique non-siliconée, l'une des deux portions constituant la chaîne principale du polymère l'autre étant greffée sur ladite chaîne principale.
Les polymères siliconés greffés utilisés dans la composition cosmétique selon l'invention sont choisis préférentiellement dans le groupe constitué par les polymères à squelette organique non siliconé greffé par des monomères contenant un polysiloxane, les polymères à squelette polysiloxanique greffé par des monomères organiques non siliconés et leurs mélanges.
Les monomères organiques non siliconés constituant la chaîne principale du polymère siliconé greffé peuvent être choisis parmi des monomères à insaturation éthylénique polymérisables par voie radicalaire, des monomères polymérisables par polycondensation tels que ceux formant des polyamides, des polyesters, des polyuréthanes, des monomères à ouverture de cycle tels que ceux du type oxazoline ou caprolactone.
Les polymères à squelette organique non-siliconé greffé par des monomères contenant un polysiloxane, conformes à l'invention, peuvent être choisis parmi ceux décrits dans les brevets US 4,693,935, US 4,728,571 et US 4,972,037 et les demandes de brevet EP-A-0 412 704, EP-A-0 412 707, EP-A-0 640 105 et WO 95/00578. Il s'agit de copolymères obtenus par polymérisation radicalaire à partir de monomères à insaturation éthylénique et de macromères

siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Le copolymère à squelette organique non-siliconé greffé par des monomères contenant un polysiloxane peut par exemple avoir la structure suivante :

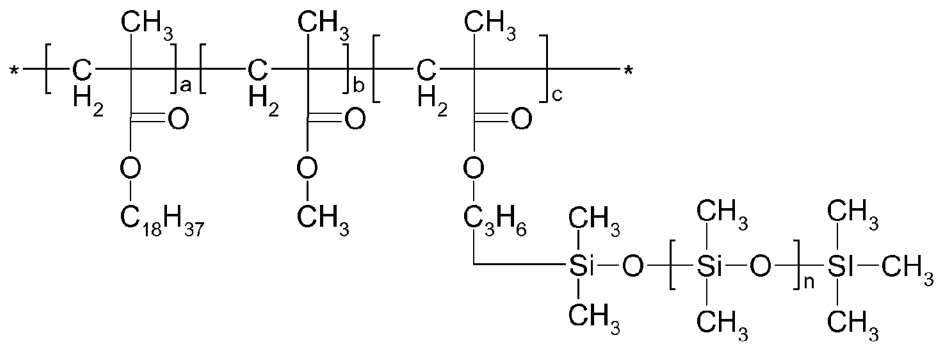

Un tel polymère est commercialisé sous le nom KP 561 commercialisés par Shin Etsu.

Le copolymère à squelette organique non-siliconé greffé par des monomères contenant un polysiloxane peut aussi avoir la structure suivante :

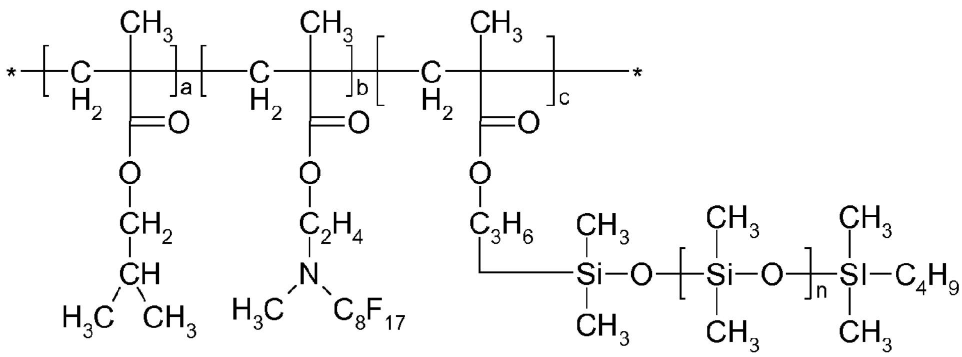

Un tel polymère, le Polysilicone 7, est commercialisé sous le nom SA70 par 3M.

D'autres copolymères à squelette organique non-siliconé greffé par des monomères contenant un polysiloxane peuvent aussi être le KP545, le KP574 et le KP575 commercialisés par Shin Etsu.

Comme composé siliconé greffé, on peut également citer le copolymère d'isobutylmethacrylate / bis-hydroxypropyl dimethicone acrylate commercialisé par Grant Industrie sous le nom Granacrysil BMAS.

Selon la présente invention, le ou les polymères siliconés greffés, à squelette polysiloxanique greffé par des monomères organiques non siliconés comprennent une chaîne principale de silicone (ou polysiloxane $(\equiv Si\text{-}O\text{-})_n$) sur laquelle se trouve greffé, à l'intérieur de ladite chaîne ainsi qu'éventuellement à l'une au moins de ses extrémités, au moins un groupement organique ne comportant pas de silicone.

Des exemples de polymères siliconés correspondant à la définition sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle. Comme composé répondant à cette définition on peut citer le poly dimethyl / methyl siloxane à groupements propyl thio-3 acrylate de methyle / methacrylate de methyle / acide methacrylique ou Polysilicone-8 commercialisé sous le nom VS80 par la société 3M.

D'autres exemples de polymères siliconés sont notamment des polydiméthylsiloxanes (PDMS) sur lesquels sont greffés, par l'intermédiaire d'un chaînon de raccordement de type thiopropylène, des motifs polymères du type poly (méth)acrylate d'isobutyle.

De préférence, la masse moléculaire en nombre des polymères siliconés à squelette polysiloxanique greffé par des monomères organiques non siliconés de l'invention varie de 10 000 à 1 000 000 environ, et encore plus préférentiellement de 10 000 à 100 000 environ.

De préférence, les polymères siliconés greffés sont choisis dans le groupe constitué par le copolymère de méthacrylate d'alkyles greffé polydiméthylsiloxane, les copolymères de méthacrylate d'isobutyle, d'acide acrylique et de macromère siliconé et le poly diméthyl / méthyl siloxane à groupements propyl thio-3-acrylate de méthyle / méthacrylate de méthyle / acide méthacrylique.

Les composés siliconés préférés sont les huiles siliconées, notamment celles décrites dans la formule (I), et les résines de silicone.

Lorsqu'ils sont présents dans la composition de l'invention, ces composés siliconés sont introduits en une quantité généralement comprise entre 0.1 % et 30% en poids, notamment entre 0.1% et 20% en poids et préférentiellement entre 0.1 et 10% en poids.

**Pigments**

Selon une variante, la composition de traitement des cheveux est une composition de coloration des fibres kératiniques qui comprend de plus des pigments. Une telle composition permet d'obtenir des gainages rémanents, colorants et ceci sans dégradation des fibres kératiniques.

Par pigment, on entend tous les pigments apportant de la couleur aux matières kératiniques. Leur solubilité dans l'eau à 25 °C et à pression atmosphérique (760 mmHg) est inférieure à 0,05 %, et de préférence inférieure à 0,01 %.

Les pigments qui peuvent être utilisés sont notamment choisis parmi les pigments organiques et / ou minéraux connus de la technique, notamment ceux qui sont décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire. Ils peuvent être enrobés ou non enrobés.

Les pigments peuvent par exemple être choisis parmi les pigments minéraux, les pigments organiques, les laques, les pigments à effets spéciaux tels que les nacres, les pigments métalliques ou les paillettes, et leurs mélanges.

Le pigment peut être un pigment minéral. Par pigment minéral, on entend tout pigment qui répond à la définition de l'encyclopédie Ullmann dans le chapitre pigment inorganique. On peut citer, parmi les pigments minéraux utiles dans la présente invention, les oxydes de fer ou de chrome, le violet de manganèse, le bleu outremer, l'hydrate de chrome et le bleu ferrique, l'oxyde de titane.

Le pigment peut-être un pigment organique. Par pigment organique, on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, isoindolinone, isoindoline, quinacridone, périnone, pérylène, dicétopyrrolopyrrole, thioindigo, dioxazine, triphénylméthane, quinophtalone.

En particulier, les pigments organiques peuvent être choisis parmi le carmin, le noir de carbone, le noir d'aniline, le jaune azo, la quinacridone, le bleu de phtalocyanine, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

Les pigments conformes à l'invention peuvent aussi être sous forme de pigments composites tels qu'ils sont décrits dans le brevet EP 1 184 426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Le pigment organique peut aussi être une laque. Par laque, on entend les colorants adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants, on peut citer le carmin de cochenille. On peut également citer les colorants connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

A titre d'exemples de laques, on peut citer le produit connu sous la dénomination suivante : D & C Red 7 (CI 15 850:1).

Le pigment peut aussi être un pigment à effets spéciaux. Par pigments à effets spéciaux, on entend les pigments qui créent d'une manière générale une apparence colorée (caractérisée par une certaine nuance, une certaine vivacité

et une certaine clarté) non uniforme et changeante en fonction des conditions d'observation (lumière, température, angles d'observation...). Ils s'opposent par-là même aux pigments colorés qui procurent une teinte uniforme opaque, semi-transparente ou transparente classique.

Il existe plusieurs types de pigments à effets spéciaux, ceux à faible indice de réfraction tels que les pigments fluorescents, photochromes ou thermochromes, et ceux à plus fort indice de réfraction tels que les nacres ou les paillettes.

A titre d'exemples de pigments à effets spéciaux, on peut citer les pigments nacrés tels que le mica recouvert d'oxyde de titane, ou d'oxychlorure de bismuth, les pigments nacrés colorés tels que le mica recouvert d'oxyde de titane et d'oxydes de fer, le mica recouvert d'oxyde de fer, le mica recouvert d'oxyde de titane et notamment de bleu ferrique ou d'oxyde de chrome, le mica recouvert d'oxyde de titane et d'un pigment organique tel que défini précédemment ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona), par la société Eckart sous la dénomination Prestige Bronze et Prestige Soft Bronze et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) et par la société Eckart sous la dénomination Prestige Copper et Prestige soft Copper; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), Dark Blue (117324) (Colorona), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

En plus des nacres sur un support mica, on peut envisager les pigments multicouches basés sur des substrats synthétiques comme l'alumine, la silice, le borosilicate de calcium et de sodium ou le borosilicate de calcium et d'aluminium, et l'aluminium.

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Le pigment peut également se présenter sous forme d'un pigment métallique. Le pigment métallique peut être choisi parmi l'argent, l'aluminium, le fer, le chrome, le nickel, le molybdène, l'or, le cuivre, le zinc, l'étain, le magnésium, l'acier, le bronze, le titane et les alliages de ces métaux. De préférence le pigment métallique est choisi parmi le cuivre, le zinc, l'aluminium, le titane, l'argent, l'or et les alliages de ces métaux. On utilise plus préférentiellement un pigment métallique choisi parmi l'aluminium (présentant avantageusement une teneur en aluminium supérieure ou égale à 99%), le cuivre (présentant avantageusement une teneur en cuivre supérieure ou égale à 95%), le bronze (présentant de préférence une teneur en cuivre allant de 70 à 95% et une teneur en zinc allant de 5 à 30%).

Le pigment métallique peut également être enrobé d'au moins une couche dite « de revêtement » d'au moins un matériau inorganique ou organique. Lorsque la particule métallique est enrobée en plus ou autrement que par le lubrifiant utilisé lors de sa production, elle est enrobée de préférence par au moins une couche d'oxyde de silicium SiO2. Ces particules métalliques enrobées de SiO2 ainsi que leur préparation sont décrites par exemple dans le document DE 10238090.

A titre de particule métallique, on peut citer des particules d'aluminium, telles que celles commercialisées sous les dénominations STARBRITE 2100 EAC® par la société SIBERLINE et METALURE® par la société ECKART. On peut également citer les poudres de bronze telles que celles commercialisées sous les dénominations Premior Super

8000 par la société Wolstenholme et sous les dénominations ROTHOFLEX, LITHOFLEX et STANDARD par la société ECKART avec par exemple les références STANDART Bronze Powder Offset 3000 Super Pale Gold (D50 3-5 μm) et LITHOFLEX XA 40-03 Rich Pale Gold (D50 3-5 μm). On peut aussi citer les particules d'alliage métallique comme des poudres de bronze enrobées de silice commercialisées sous la dénomination de VISIONAIRE Honey (taille 5-50 μm) et sous la dénomination de VISIONAIRE Amber (taille 5-50 μm) par la société Eckart ainsi que celles commercialisées sous la dénomination DOROLAN 08/0 Pale Gold (D50 7-9 μm), la poudre d'aluminium enrobée de SiO2 de commercialisée sous la référence VISIONAIRE Silver Sea (taille 5-50 μm) et les poudres de cuivre enrobée de SiO2 commercialisée sous la référence VISIONAIRE Cinnamon (taille 5-50 μm) et sous la référence VISIONAIRE Lava (taille 5-50 μm) par la société ECKART ainsi que celles commercialisées sous la dénomination DOROLAN 10/0 Copper (D50 9-11 μm).

La variété des pigments qui peuvent être utilisés dans la présente invention permet d'obtenir une riche palette de couleurs, ainsi que des effets optiques particuliers tels que des effets métalliques, interférentiels.

La taille du pigment utilisé dans la composition cosmétique selon la présente invention est généralement comprise entre 10 nm et 200 μm, de préférence entre 20 nm et 80 μm, et plus préférentiellement entre 30 nm et 50 μm.

Les pigments peuvent être dispersés dans le produit grâce à un agent dispersant.

L'agent dispersant sert à protéger les particules dispersées contre leur agglomération ou floculation. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en $C_8$ à $C_{20}$ et de polyol comme le glycérol, la diglycérine, tel que le stéarate d'acide poly(12-hydroxystéarique) de poids moléculaire d'environ 750 g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans les compositions de l'invention, on peut citer les dérivés ammonium quaternaire d'acides gras polycondensés comme le Solsperse 17 000 vendu par la société Avecia, les mélanges de polydiméthylsiloxane / oxypropylène tels que ceux vendus par la société Dow Corning sous les références DC2-5185, DC2-5225 C.

Les pigments utilisés dans la composition cosmétique selon l'invention peuvent être traités en surface par un agent organique.

Ainsi les pigments préalablement traités en surface utiles dans le cadre de l'invention sont des pigments qui ont subi totalement ou partiellement un traitement de surface de nature chimique, électronique, électro-chimique, mécano-chimique ou mécanique, avec un agent organique tel que ceux qui sont décrits notamment dans Cosmetics and Toiletries, Février 1990, Vol. 105, p. 53-64 avant d'être dispersés dans la composition conforme à l'invention. Ces agents organiques peuvent être par exemple choisis parmi les acides aminés ; les cires, par exemple la cire de carnauba et la cire d'abeille ; les acides gras, les alcools gras et leurs dérivés, tels que l'acide stéarique, l'acide hydroxystéarique, l'alcool stéarylique, l'alcool hydroxystéarylique, l'acide laurique et leurs dérivés ; les tensio-actifs anioniques ; les lécithines ; les sels de sodium, potassium, magnésium, fer, titane, zinc ou aluminium d'acides gras, par exemple le stéarate ou laurate d'aluminium ; les alcoxydes métalliques ; les polysaccharides, par exemple le chitosane, la cellulose et ses dérivés ; le polyéthylène ; les polymères (méth)acryliques, par exemple les polyméthylmethacrylates ; les polymères et copolymères contenant des motifs acrylates ; les protéines ; les alcanoamines ; les composés siliconés, par exemple les silicones, les polydiméthylsiloxanes, les alcoxysilanes, les alkylsilanes, les siloxy-silicates ; les composés organiques fluorés, par exemple les perfluoroalkyle éthers ; les composés fluoro-siliconés.

Les pigments traités en surface utiles dans la composition cosmétique selon l'invention peuvent aussi avoir été traités par un mélange de ces composés et / ou avoir subi plusieurs traitements de surface.

Les pigments traités en surface utiles dans le cadre de la présente invention peuvent être préparés selon des techniques de traitement de surface bien connues de l'homme de l'art ou trouvés tels quels dans le commerce.

De préférence, les pigments traités en surface sont recouverts par une couche organique.

L'agent organique avec lequel sont traités les pigments peut être déposé sur les pigments par évaporation de solvant, réaction chimique entre les molécules de l'agent de surface ou création d'une liaison covalente entre l'agent de surface et les pigments.

Le traitement en surface peut ainsi être réalisé par exemple par réaction chimique d'un agent de surface avec la surface des pigments et création d'une liaison covalente entre l'agent de surface et les pigments ou les charges. Cette méthode est notamment décrite dans le brevet US 4 578 266.

De préférence, on utilisera un agent organique lié aux pigments de manière covalente.

L'agent pour le traitement de surface peut représenter de 0,1 à 50% en poids du poids total des pigments traités en surface, de préférence de 0,5 à 30% en poids, et encore plus préférentiellement de 1 à 10% en poids.

Lorsqu'ils sont présents, la quantité de pigments est généralement comprise entre 0,1 à 40% en poids, de préférence 0,5 à 20% en poids du poids total de la composition.

***Autres additifs***

Lorsque le polymère a une température de transition vitreuse trop élevée pour l'utilisation désirée, on peut y associer un plastifiant de façon à abaisser cette température du mélange utilisé. Le plastifiant peut être choisi parmi les plastifiants utilisés habituellement dans le domaine d'application, et notamment parmi les composés pouvant être des solvants pour le polymère.

De préférence, le plastifiant a une masse moléculaire inférieure ou égale à 5000 g/mol, de préférence inférieure ou égale à 2000 g/mol, préférentiellement inférieure ou égale à 1000 g/mol. Le plastifiant a avantageusement une masse moléculaire supérieure ou égale à 100 g/mol.

Ainsi, la composition peut comprendre, en outre, au moins un agent plastifiant. En particulier, on peut citer, seuls ou en mélange, les plastifiants usuels, tels que :

- les glycols et leurs dérivés, tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les polyéthylène glycols, les polypropylène glycols, les copolymères polyéthylène glycols-polypropylène glycols et leurs mélanges, notamment les polypropylène glycols de haut poids moléculaire, ayant par exemple une masse moléculaire allant de 500 à 15000, tels que par exemple
- les esters de glycol ;
- des esters d'acides, notamment carboxyliques, tels que des citrates, des phtalates, des adipates, des carbonates, de tartrates, des phosphates, des sébaçates ;
- les esters issus de la réaction d'un acide monocarboxylique de formule $R_{11}COOH$ avec un diol de formule $HOR_{12}OH$ avec $R_{11}$ et $R_{12}$, identiques ou différents, représentent une chaîne hydrocarbonée, comprenant de préférence de 3 à 15 atomes de carbone, linéaire, ramifiée ou cyclique, saturée ou insaturée comprenant éventuellement un ou plusieurs hétéroatomes tels que N, O , S, en particulier le monoester résultant de la réaction de l'acide isobutyrique et d'octanediol tel que le triméthyl-2,2,4-pentane-diol-1,3, tel que celui commercialisé sous la référence TEXANOL Ester Alcohol par la société Eastman Chemical,
- des dérivés oxyéthylénés, tels que les huiles oxyéthylénées, notamment les huiles végétales, telles que l'huile de ricin ;
- leurs mélanges.

La composition selon l'invention peut comprendre un ou plusieurs agents épaississants choisis parmi les agents épaississants polymériques, les agents épaississants minéraux et leurs mélanges.

L'épaississant peut être minéral ou organique, polymère ou non polymère. L'épaississant peut être choisi pour épaissir une phase aqueuse ou une phase grasse de la composition selon les cas.

On entend par épaississant, un composé qui modifie la rhéologie du milieu dans lequel il est incorporé en accroissant d'au moins 100 cps la viscosité du milieu à 25 °C et à un taux de cisaillement de 1 $s^{-1}$. Cette viscosité peut être mesurée à l'aide d'un viscosimètre cône/plan (Rhéomètre Haake R600 ou analogue).

L'épaississant de milieu aqueux peut être choisi parmi :

- les argiles hydrophiles,
- la silice pyrogénée hydrophile,
- les épaississants cellulosiques hydrosolubles, tels que l'hydroxyethylcellulose, la méthylcellulose, l'hydroxypropylcellulose. Parmi celles-ci on peut citer notamment les gommes vendues sous la dénomination Cellolize QP 4400 H par a société Amercol.
- les gommes de guar non ionique comprenant des groupements hydroxyalkyle en $C_1$-$C_6$. On peut mentionner à titre d'exemple les groupements hydroxymethyle, hydroxypropyle et hydroxybutyle. De telles gommes de guar sont notamment vendue sous les dénominations commerciales JAGUAR HP8, JAGUAR HP60, JAGUAR HP120, JAGUAR HP105 par la société MEYHALL ou sous la dénomination GALACTASOL 40H4FD2 par la société AQUALON.
- les carraghénanes,
- les gommes de caroube, de scléroglucane, de gellane, de rhamsan, de karaya,
- les alginates, les maltodextrines, l'amidon et ses dérivés, l'acide hyaluronique et ses sels,
- les polymères poly(métha)crylates de glycéryle vendus sous les dénominations de "Hispagel" ou "Lubragel" par les Sociétés Hispano Quimica ou Guardian,
- l'alcool polyvinylique,

- les polymères et les copolymères réticulés d'acrylamide, tels que ceux vendus sous les dénominations de "PAS 5161" ou "Bozepol C" par la Société Hoechst, de "Sepigel 305" par la Société Seppic par la Société Allied Colloïd, ou encore,
- les homopolymères réticulés de chlorure de méthacryloyloxyéthyltriméthylammonium vendus sous la dénomination de "Salcare SC95" par la Société Allied Colloïd,
- les polymères associatifs et notamment les polyuréthanes associatifs.

De tels épaississants sont notamment décrits dans les demandes EP-A-1400234, dont le contenu est incorporé à titre de référence.

L'épaississant de milieu huileux peut être choisi parmi

- les argiles organophiles,
- les silices pyrogénées hydrophobes
- les gommes de guar alkylées (avec groupe alkyle en C1-C6), telles que celles décrites dans EP-A-708114 ;
- les polymères gélifiant d'huile comme les polymères tribloc ou en étoile résultant de la polymérisation ou copolymérisation d'au moins monomère à groupe éthylénique, comme les polymères vendus sous la dénomination Kraton ;
- les polymères de masse moléculaire moyenne en poids inférieure à 100 000, comportant a) un squelette polymérique ayant des motifs de répétition hydrocarbonés pourvus d'au moins un hétéroatome, et éventuellement b) au moins une chaîne grasse pendante et / ou au moins une chaîne grasse terminale éventuellement fonctionnalisées, ayant de 6 à 120 atomes de carbone et étant liées à ces motifs hydrocarbonés, telles que décrites dans les demandes WO-A-02/056847, WO-A-02/47619 dont le contenu est incorporé à titre de référence ; en particulier les résines de polyamides (notamment comprenant des groupes alkyles ayant de 12 à 22 atomes de carbone) telles que celles décrites dans US-A-5783657 dont le contenu est incorporé à titre de référence.
- les résines de polyamides siliconées telles que décrites dans la demande EP-A-1266647, dans la demande de brevet français déposée sous le n° 0 216 039 dont le contenu est incorporé à titre de référence.

De tels épaississants sont notamment décrits dans les demandes EP-A-1400234, dont le contenu est incorporé à titre de référence.

L'épaississant peut être un agent gélifiant organique, c'est-à-dire un agent comprenant au moins un composé organique. Les organogélateurs peuvent être choisis parmi ceux décrits dans la demande WO-A-03/105788 dont le contenu est incorporé à titre de référence.

Plus précisément, l'agent épaississant polymérique présent dans la composition selon l'invention est un polymère amorphe formé par polymérisation d'une oléfine. L'oléfine peut être notamment un monomère à insaturation éthylénique élastomérique.

Comme exemple d'oléfine, on peut citer les monomères de carbure éthylénique, ayant notamment une ou deux insaturations éthylénique, ayant de 2 à 5 atomes de carbone tels que l'éthylène, le propylène, le butadiène, l'isoprène.

L'agent épaississant polymérique est capable d'épaissir ou de gélifier la composition. Par polymère amorphe, on entend un polymère qui n'a pas de forme cristalline. L'agent épaississant polymérique peut être également filmogène.

L'agent épaississant polymérique peut être notamment un copolymère dibloc, tribloc, multibloc, radial, étoile, ou leurs mélanges.

De tels agents épaississants polymériques sont décrits dans la demande US-A-2002/005562 et dans le brevet US-A-5 221 534

Avantageusement, l'agent épaississant polymérique est un copolymère bloc amorphe de styrène et d'oléfine.

L'agent épaississant polymérique est de préférence hydrogéné pour réduire les insaturations éthyléniques résiduelles après la polymérisation des monomères.

En particulier, l'agent épaississant polymérique est un copolymère, éventuellement hydrogéné, à blocs styrène et à blocs éthylène/alkylène en C3-C4.

Comme copolymère dibloc, de préférence hydrogéné, on peut citer les copolymères de styrène-éthylène/propylène, les copolymères de styrène-éthylène/butadiène. Des polymères diblocs sont notamment vendus sous la dénomination Kraton® G1701 E par la société Kraton Polymers.

Comme copolymère tribloc, de préférence hydrogéné, on peut citer les copolymères de styrène-éthylène/propylène-styrène, les copolymères de styrène-éthylène/butadiène-styrène, les copolymères de styrène-isoprène-styrène, les copolymères de styrène-butadiène-styrène. Des polymères triblocs sont notamment vendus sous les dénominations Kraton® G1650, Kraton® G1652, Kraton® D1101, Kraton® D1102, Kraton® D1160 par la société Kraton Polymers.

On peut également utiliser un mélange de copolymère hydrogéné tribloc styrène-butylène / éthylène-styrène et de polymère étoile hydrogéné éthylène-propylène-styrène, un tel mélange étant notamment dans l'isododécane. De

tels mélanges sont par exemple vendus par la société PENRECO sous les dénominations commerciales VERSAGEL® M5960 et VERSAGEL® M5670.

Avantageusement, on utilise comme agent épaississant polymérique un copolymère dibloc tel que ceux décrits précédemment, en particulier un copolymère dibloc de styrène-éthylène/propylène.

Plus précisément, les argiles organophiles sont des argiles modifiées par des composés chimiques rendant l'argile apte à gonfler.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson", dont l'enseignement est ici inclus à titre de référence.

Les argiles sont des silicates contenant un cation pouvant être choisi parmi les cations de calcium, de magnésium, d'aluminium, de sodium, de potassium, de lithium et leurs mélanges.

A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.

Ces argiles peuvent être d'origine naturelle ou synthétique. De préférence, on utilise les argiles qui sont cosmétiquement compatibles et acceptables avec les matières kératiniques.

L'argile organophile peut être choisie parmi la montmorrilonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leurs surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :

- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

De préférence, on utilise comme agent épaississant minéral une hectorite ou une bentonite organomodifiée.

Le ou les agents épaississants peuvent être présents dans la composition en une teneur totale allant de 0,1 à 10% en poids par rapport au poids total de la composition, de préférence allant de 0,5 à 7% en poids, et plus préférentiellement allant de 0,5 à 5% en poids.

La composition conforme à l'invention peut également contenir un ou plusieurs agents utilisés habituellement en cosmétique, choisi, par exemple, parmi des agents réducteurs, des corps gras, des adoucissants, des agents antimousse, des agents hydratants, des filtres UV, des colloïdes minéraux, des peptisants, des parfums, des tensio-actifs anioniques, cationiques, non ioniques ou amphotères, des protéines, des vitamines, des propulseurs, les cires oxyéthylénées ou non, les paraffines, les acides gras en $C_{10}$-$C_{30}$ tels que l'acide stéarique, l'acide laurique, les amides gras en $C_{10}$-$C_{30}$ tel que le diéthanolamide laurique, des polymères anioniques, cationiques, non ioniques ou amphotères.

Les additifs ci-dessus sont en général présents en quantité comprise pour chacun d'eux entre 0,01 et 20% en poids par rapport au poids de la composition.

Bien entendu, l'homme de l'art veillera à choisir ce ou ces éventuels additifs de manière telle que les propriétés avantageuses attachées intrinsèquement à la formation du gainage conforme à l'invention ne soient pas, ou substantiellement pas, altérées.

La composition selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de mousse, de stick, de dispersion de vésicules notamment de lipides ioniques ou non, de lotion biphase ou multiphase, de spray, de poudre, de pâte. La composition peut également se présenter sous forme de laque.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition peut être une composition anhydre, c'est-à-dire une composition contenant moins de 2% en poids d'eau, voire moins de 0,5% d'eau, notamment exempte d'eau, l'eau n'étant pas ajoutée lors de la préparation de la composition mais correspondant à l'eau résiduelle apportée par les ingrédients mélangés.

La composition décrite ci-dessus peut être mise en oeuvre sur cheveux secs ou humides ainsi que sur tous types de cheveux clairs ou foncés, naturels ou colorés, permanentés, décolorés ou défrisés.

Selon un mode de réalisation particulier du procédé de l'invention, les cheveux sont lavés avant application de la composition décrite ci-dessus. De préférence, l'application de la composition se fait sur cheveux propres.

L'application peut se faire sur des cheveux secs ou humides.

L'application sur les cheveux peut être mise en oeuvre par exemple au moyen d'un peigne, d'un pinceau à l'aide d'une brosse ou aux doigts.

Selon un mode de réalisation particulier de l'invention, l'application de la composition est ensuite suivie d'un séchage à une température supérieure à 40°C. De préférence, cette température est supérieure à 45°C. Encore plus préférentiellement, cette température est supérieure à 45°C et inférieure à 220°C.

Le séchage peut être réalisé immédiatement après l'application ou après un temps de pose pouvant aller de 1 minute à 30 minutes.

De préférence les cheveux sont séchés, en plus d'un apport de chaleur, avec un flux d'air. Ce flux d'air pendant le séchage permet d'améliorer l'individualisation du gainage.

Durant le séchage, une action mécanique sur les mèches peut être exercée telle qu'un peignage, un brossage, le passage des doigts.

L'étape de séchage du procédé de l'invention peut être mise en oeuvre avec un casque, un sèche-cheveux, un fer à lisser...

Lorsque l'étape de séchage est mise en oeuvre avec un casque ou un sèche-cheveux, la température du séchage est comprise entre 40 et 110 degrés, de préférence entre 50 et 90 degrés.

Lorsque l'étape de séchage est mise en oeuvre avec un fer à lisser, la température du séchage est comprise entre 110 et 220 degrés, de préférence entre 140 et 200 degrés.

Une fois le séchage terminé, un rinçage ou un shampooing terminal peut éventuellement être réalisé.

Les exemples qui suivent servent à illustrer l'invention.

**Exemple 1 : Composition de traitement :**

Les compositions suivantes sont réalisées :

| Composition | A | A' (hors invention) |
|---|---|---|
| Polymère supramoléculaire obtenu à partir de GI2000 à titre Polymère supra moléculaire obtenu à partir de GI2000 à titre de polyalcène fonctionnalisé de formule A et d'un greffon de formule B dans lequel L désigne un radical isophorone à 25% dans l'isododécane preparé comme décrit ci-dessous | 40 g | 40 g |
| Poly(methacrylate d'isobornyle-co-acrylate d'isobornyle-co-acrylate d'isobutyle-co-acide acrylique à 50% dans l'isododécane preparé comme décrit ci-dessous8-preparé comme décrit ci-dessous *Résine Triméthylsiloxysilicate commercialisée par Momentive Performance Materials sous le nom SR1000 | 8 g | - |

(suite)

| Composition | A | A' (hors invention) |
|---|---|---|
| Résine Triméthylsiloxysilicate commercialisée par Momentive Performance Materials sous le nom SR1000 | 1.5 g | 1.5 g |
| Ethanol | 5 g | 5 g |
| Isododécane | Qs 100 g | Qs 100 g |

0,5 g de la composition A est appliqué sur une mèche de 2.5g de cheveux de hauteur de ton 4 propres et humides. Après 2 minutes de pause, la mèche est séchée au sèche-cheveux à une température de 80°C pendant 2 minutes. On obtient une mèche dont les cheveux sont individualisés et corporisés, la corporisation obtenue est rémanente au shampooing.
Une mèche appliquée avec la composition A' possède une résistance corps gras, notamment au sébum, inférieureà une mèche appliquée avec la composition A.

**Exemple 2 : Composition de traitement :**

La composition suivante est réalisée :

| Composition | B |
|---|---|
| Polymère supramoléculaire obtenu à partir de GI2000 à titre de polyalcène fonctionnalisé de formule A et d'un greffon de formule B dans lequel L désigne un radical isophorone à 25% dans l'isododécane preparé comme décrit ci-dessous * | 40 g |
| Mélange Polydimethylsiloxane alpha-omega dihydroxyle / Cyclopentadimethylsiloxane (14,7/85,3) commercialisé par Dow Corning sous le nom DC1501 Fluid | 14 g |
| Poly(methacrylate d'isobornyle-co-acrylate d'isobornyle-co-acrylate d'isobutyle-co-acide acrylique à 50% dans l'isododécane preparé comme décrit ci-dessous * | 8 g |
| Résine Triméthylsiloxysilicate commercialisée par Momentive Performance Materials sous le nom SR1000 | 1.5 g |
| Ethanol | 5 g |
| Isododécane | Qs 100 g |
| * la concentration indiquée correspond au polymère pur. | |

0,5 g de la composition B est appliqué sur une mèche de 2.5 g de cheveux de hauteur de ton 4 propres et humides. Après 2 minutes de pause, la mèche est séchée au sèche-cheveux à une température de 80°C pendant 2 minutes. On obtient une mèche dont les cheveux sont individualisés et corporisés, la corporisation obtenue est rémanente au shampooing. Cette mèche possède également une bonne résistance aux corps gras.

**Exemple 3 : Composition de coloration :**

Les compositions suivantes sont réalisées :

| Composition | C | C' (hors invention) |
|---|---|---|
| Polymère supramoléculaire obtenu à partir de GI2000 à titre de de polyalcène fonctionnalisé de formule A et d'un greffon de formule B dans lequel L désigne un radical isophorone à 25% dans l'isododécane preparé comme décrit ci-dessous * | 32 g | 32 g |
| Mélange Polydimethylsiloxane alpha-omega dihydroxyle/ Cyclopentadimethylsiloxane (14,7/85,3) commercialisé par Dow Corning sous le nom DC1501 Fluid | 14 g | 14 g |

(suite)

| Composition | C | C' (hors invention) |
|---|---|---|
| Nacre mica enrobé d'oxyde de fer brun commercialisée par Eckart sous le nom Prestige Soft Bronze | 8 g | 8 g |
| Poly(methacrylate d'isobornyle-co-acrylate d'isobornyle-co-acrylate d'isobutyle-co-acide acrylique à 50% dans l'isododécane preparé comme décrit ci-dessous * | 8 g | - |
| Ethanol | 5 g | 5 g |
| Isododécane | Qs 100 g | Qs 100 g |

0,6 g de la composition C est appliqué sur une mèche de 1g de cheveux de hauteur de ton 4 propres et humides. Après 2 minutes de pause, la mèche est séchée au sèche-cheveux à une température de 80°C pendant 2 minutes. On obtient une mèche colorée dont les cheveux sont individualisés et dont la couleur est très homogène et rémanente au shampooing.
Une mèche appliquée avec la composition C' possède une résistance aux shampooings et une résistance aux corps gras, notamment au sébum, inférieures qu'une mèche appliquée avec la composition C.

**Exemple 4 :**

Les compositions suivantes sont réalisées :

| Composition | D | D' |
|---|---|---|
| Polymère supramoléculaire obtenu à partir de GI3000 à titre de de polyalcène fonctionnalisé de formule A et d'un greffon de formule B dans lequel L désigne un radical hexaméthylène à 25% dans l'isododécane preparé comme décrit ci-dessous * | 32 g | - |
| Polymère supramoléculaire obtenu à partir de GI2000 à titre de de polyalcène fonctionnalisé de formule A et d'un greffon de formule B dans lequel L désigne un radical isophorone à 25% dans l'isododécane preparé comme décrit ci-dessous * | - | 32 g |
| Mélange Polydimethylsiloxane alpha-omega dihydroxyle/Cyclopentadimethylsiloxane (14,7 / 85,3) commercialisé par Dow Corning sous le nom DC1501 Fluid | 14 g | 14 g |
| Nacre mica enrobé d'oxyde de fer brun commercialisée par Eckart sous le nom Prestige Soft Bronze | 8 g | 8 g |
| Poly(methacrylate d'isobornyle-co-acrylate d'isobornyle-co-acrylate d'isobutyle-co-acide acrylique à 50% dans l'isododécane preparé comme décrit ci-dessous * | 8 g | 8 g |
| Polyméthylsilsesquioxane commercialisé sous le nom Wacker Belsil PMS MK Powder par la société Wacker | 3 g | 3 g |
| Résine Triméthylsiloxysilicate commercialisée par Momentive Performance Materials sous le nom SR1000 | 2 g | 2 g |
| Ethanol | 5 g | 5 g |
| Isododécane | Qs 100 g | Qs 100 g |

0,6 g de la composition D ou D' est appliqué sur une mèche de 1g de cheveux de hauteur de ton 4 propres et humides. Après 2 minutes de pause, la mèche est séchée au sèche-cheveux à une température de 80°C pendant 2 minutes. On obtient une mèche colorée dont les cheveux sont individualisés et dont la couleur est très homogène et rémanente au shampooing.
Le polymère supramoléculaire utilisé dans les exemples 1, 2, 3 et 4 (Composition D') ci-dessus est synthétisé de la manière suivante.
106,1 g de polymère GI2000 commercialisé par la société NISSO, en présence de 22 mg de catalyseur, le dilaurate de dibutyl étain, sont chauffés à 80°C, sous vide pendant 2 heures. La température du mélange est descendue à 20°C, sous argon, suivi de l'addition de 10 mL d'isododécane. 19,3 g d'isophorone diisocyanate sont additionnés. Le mélange est agité pendant 16 heures à 20°C, sous atmosphère contrôlée, puis est chauffé à 120°C, suivi de l'addition de 25 mL

de propylène carbonate. 12 g de 6-méthyl isocytosine sont additionnés. Il en résulte une suspension homogène blanche. Cette suspension est chauffée à 140°C et est agitée à cette température pendant 6 heures. La réaction est suivie par spectroscopie infra-rouge, jusqu'à la disparition totale du pic caractéristique des isocyanates (2250 cm-1). Le mélange est ensuite redescendu à 30°C, et 400 mL d'heptane, 200 mL de THF et 50 mL d'éthanol sont additionnés au mélange, avant filtration sur célite. Un stripping à l'isododécane, permet d'obtenir le polymère à 20 % d'extrait sec. Le polymère est caractérisé par GPC (Mn = 7000 avec un Ip de 2,05).

Le polymère supramoléculaire utilisé dans l'exemple 4 (Composition D) ci-dessus est synthétisé de la manière suivante. 100 g de polymère de GI3000 commercialisé par la société NISSO sont séchés à 80°C, sous vide pendant une nuit. Ce polymère est mis en solution dans 400 mL de toluène anhydre. 25 μL de catalyseur, le dilaurate de dibutyl étain sont additionnés au mélange réactionnel. Le milieu est chauffé à 80°C, et mélangé jusqu'à obtenir une solution homogène. 15 g de molécule fonctionnalisée isocyanate de structure suivante :

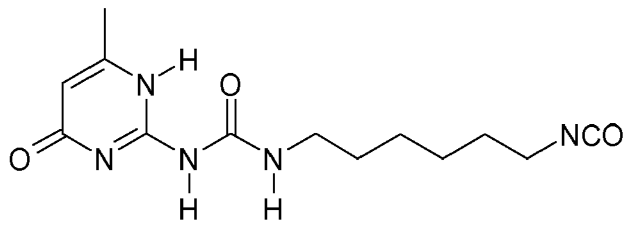

sont additionnés en solution dans 300 mL de toluène anhydre, sous atmosphère contrôlée à 40°C. Le mélange réactionnel est chauffé à 100 °C et agité à cette température pendant 4 heures. Le suivi de la réaction est fait par spectroscopie infra rouge, avec le suivi de la disparition totale du pic caractéristique des isocyanate à 2260 cm-1. A la fin de la réaction, 100 mL d'éthanol sont ajoutés pour éliminer toute trace d'isocyanate résiduel. Le mélange est filtré après avoir rajouté de l'isododécane pour rendre la solution moins visqueuse. La solution de polymère est alors directement strippée à l'isododécane. Le polymère final est obtenu à 21% d'extrait sec dans l'isododécane et est caractérisé par GPC (Mn = 6400 et un indice de polydispersité Ip de 1,85) et RMN 1 H (spectre conforme à ce qui est attendu).

Le copolymère de poly(acrylate d'isobornyle/méthacrylate d'isobornyle /acrylate isobutyle/acide acrylique) utilisé dans les exemples est synthétisé selon le mode opératoire suivant

300 g d'isododécane sont introduits dans un réacteur de 1 litre, puis on augmente la température de façon à passer de la température ambiante (25°C) à 90°C en 1 heure.

On ajoute ensuite, à 90°C et en 1 heure, 105 g de méthacrylate d'isobornyle (fabriqué par Arkema), 105 g d'acrylate d'isobornyle (fabriqué par Arkema) et 1,8 g de 2.5- Bis(2-éthylhexanoylperoxy)-2.5-diméthylhexane (Trigonox® 141 d'Akzo Nobel).

Le mélange est maintenu 1 h 30 à 90°C.

On introduit ensuite au mélange précédent, toujours à 90°C et en 30 minutes, 75 g d'acrylate d'isobutyle (fabriqué par Fluka), 15 g d'acide acrylique et 1,2 g de 2.5- Bis(2-éthylhexanoylperoxy)-2.5-diméthylhexane.

Le mélange est maintenu 3 heures à 90°C, puis l'ensemble est refroidi.

On obtient une solution à 50% de matière active en polymère dans l'isododécane.

On obtient un polymère comprenant une première séquence rigide poly(acrylate d'isobornyle/méthacrylate d'isobornyle) ayant une Tg de 110°C, une deuxième séquence souple poly(acrylate d'isobutyle/acide acrylique) ayant une Tg de -9°C et une séquence intermédiaire qui est un polymère statistique acrylate d'isobornyle/méthacrylate d'isobornyle/acrylate d'isobutyle/acide acrylique.

## Revendications

1. Composition de traitement des fibres kératiniques comprenant un ou plusieurs polymères supramoléculaires à base de polyalcène,
   un ou plusieurs un ou plusieurs copolymères éthyléniques séquencés, contenant au moins une première séquence ayant une température de transition vitreuse (Tg) supérieure ou égale à 40°C et étant issue en totalité ou en partie de un ou plusieurs premiers monomères, qui sont tels que l'homopolymère préparé à partir de ces monomères a une température de transition vitreuse supérieure ou égale à 40°C, et au moins une deuxième séquence ayant une température de transition vitreuse inférieure ou égale à 20°C et étant issue en totalité ou en partie de un ou plusieurs deuxièmes monomères, qui sont tels que l'homopolymère préparé à partir de ces monomères a une température de transition vitreuse inférieure ou égale à 20°C, ladite première séquence et ladite deuxième séquence étant reliées entre elles par un segment intermédiaire statistique comprenant au moins un desdits premiers monomères constitutifs de la première séquence et au moins un desdits deuxièmes monomères constitutifs de la deuxième séquence, et ledit copolymère séquencé ayant un indice de polydispersité I supérieur à 2, et
un ou plusieurs solvants volatils.

**2.** Composition selon la revendication 1 dans laquelle le polyalcène est choisi parmi les poly(éthylènebutylène), les polybutadiènes hydrogénés ou non et les polyisoprènes.

**3.** Composition selon les revendications 1 ou 2 dans laquelle le ou les polymères supramoléculaires à base de polyalcène sont issus de la condensation d'au moins un polymère polyalcène fonctionnalisé par au moins un groupe réactif, avec au moins un greffon fonctionnalisé par au moins un groupe réactif susceptible de réagir avec le ou les groupes réactifs du polymère polyalcène fonctionnalisé, le dit greffon étant porteur d'au moins un groupe capable de former au moins 3 liaisons H.

**4.** Composition selon la revendication 3 dans laquelle le polyalcène fonctionnalisé est de formule A :

HX-R-X'H

XH et X'H étant des groupes réactifs, avec X et X', identiques ou différents, choisis parmi O, S, NH, ou $NR_a$, $R_a$ représentant un groupe alkyle en $C_1$-$C_6$ ;
R représentant un homo ou un copolymère issu d'un ou plusieurs alcènes mono ou polyinsaturés en $C_2$-$C_{10}$.

**5.** Composition selon l'une quelconque des revendications 1 à 3 dans laquelle le polyalcène fonctionnalisé est choisi parmi les poly(éthylènebutylène) à extrémités hydroxyles, les polybutadiènes hydrogénés ou non à extrémités hydroxyles, et les polyisoprènes à extrémités hydroxyles.

**6.** Composition selon l'une quelconque des revendications 3 à 4 dans laquelle le greffon possède au moins un groupe uréidopyrimidone.

**7.** Composition selon l'une quelconque des revendications 3 à 5 dans laquelle le ou les groupes réactifs du greffon sont des groupes isocyanates.

**8.** Composition selon l'une quelconque des revendications 3 à 6 dans laquelle le greffon est de formule (B) :

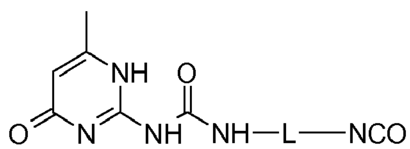
(B)

L désignant un groupement : phény!ène; 1,4-nitrophényle ; 1,2-éthylène ; 1,6-héxylène ; 1,4-butylène; 1,6-(2,4,4-triméthylhexylène) ; 1,4-(4-méthylpentylène) ; 1,5-(5-méthylhexylène) ; 1,6-(6-méthylheptylène) ; 1,5-(2,2,5-triméthylhexylène) ; 1,7-(3,7-diméthyloctylène) ; -isophorone- ; 4,4'-méthylène biscyclohexylène ; tolylène ; 2-méthyl-1,3-phénylène ; 4-méthyl-1,3-phénylène ; 4,4-biphénylèneméthylène.

**9.** Composition selon les revendications 1 ou 2 dans laquelle le ou les polymère supramoléculaires à base de polyalcène sont obtenus à partir d'un polymère (A1) comportant une partie polyalcène, le dit polymère étant fonctionnalisé par au moins un groupe réactif (B1), par condensation avec au moins une molécule (A3) comportant au moins un groupe réactif (B2), la dite molécule étant tel qu'après réaction des groupes (B1) et (B2) il y ait formation d'une entité capable de former au moins 3 liaisons H, de préférence au moins 4 liaisons H.

**10.** Composition selon la revendication 9 dans laquelle les polymères (A1) sont de formule (C1) :

CON-L-NCO-X-R- X'-CON-L-NCO

(C1)
dans laquelle avec X et X', identiques ou différents, choisis parmi O, S, NH, ou $NR_a$, $R_a$ représentant un groupe alkyle en $C_1$-$C_6$ ;

R représentant un homo ou un copolymère issu d'un ou plusieurs alcènes mono ou polyinsaturés en $C_2$-$C_{10}$ ; L désignant un groupement : phénylène ; 1,4-nitrophényle ; 1,2-éthylène ; 1,6-héxylène ; 1,4-butylène ; 1,6-(2,4,4-triméthylhexylène) ; 1,4-(4-méthylpentylène) ; 1,5-(5-méthylhexylène) ; 1,6-(6-méthylheptylène) ; 1,5-(2,2,5-triméthylhexylène) ; 1,7-(3,7-diméthyloctylène) ; -isophorone- ; 4,4'-méthylène biscyclohexylène ; tolylène ; 2-méthyl-1,3-phénylène ; 4-méthyl-1,3-phénylène ; 4,4-biphénylèneméthylène.

**11.** Composition selon la revendication 9 dans laquelle la molécule (A3) est la 6 méthylisocytosine de formule :

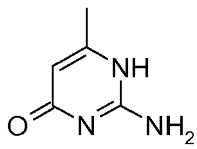

**12.** Composition selon l'une quelconque des revendications précédentes dans laquelle le polymère supramoléculaire de l'invention est de formule C :

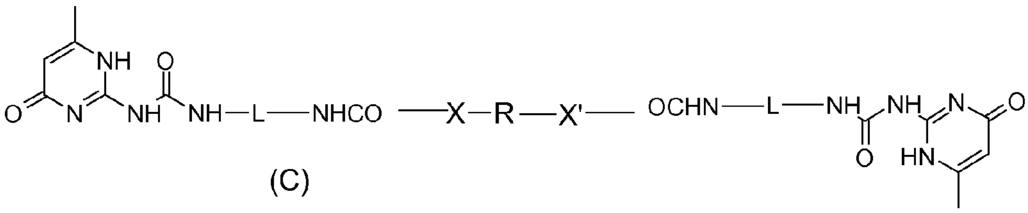

(C)

avec X et X', identiques ou différents, choisis parmi O, S, NH, ou $NR_a$, $R_a$ représentant un groupe alkyle en $C_1$-$C_6$ ; R représentant un homo ou un copolymère issu d'un ou plusieurs alcènes mono ou polyinsaturés en $C_2$-$C_{10}$ ; et L désignant un groupement : phénylène ; 1,4-nitrophényle ; 1,2-éthylène ; 1,6-héxylène ; 1,4-butylène ; 1,6-(2,4,4-triméthylhexylène) ; 1,4-(4-méthylpentylène) ; 1,5-(5-méthylhexylène) ; 1,6-(6-méthylheptylène) ; 1,5-(2,2,5-triméthylhexylène) ; 1,7-(3,7-diméthyloctylène) ; -isophorone- ; 4,4'-méthylène biscyclohexylène ; tolylène ; 2-méthyl-1,3-phénylène ; 4-méthyl-1,3-phénylène ; 4,4-biphénylèneméthylène.

**13.** Composition selon l'une quelconque des revendications précédentes, **caractérisée en ce que** le copolymère éthyléniques séquencé comprend une première séquence obtenu à partir du ou des premiers monomères, qui sont tels que l'homopolymère préparé à partir de ces monomères a une températures de transition vitreuse supérieure ou égale à 40°C, sont choisis parmi :

- les méthacrylates de formule CH2 = C(CH3)-COOR1
dans laquelle $R_1$ représente un groupe alkyle non substitué, linéaire ou ramifié, contenant de 1 à 4 atomes de carbone, un groupe cycloalkyle $C_4$ à $C_{12}$,
- les acrylates de formule $CH_2 = CH\text{-}COOR_2$
dans laquelle $R_2$ représente un groupe cycloalkyle en $C_4$ à $C_{12}$,
- les (méth)acrylamides de formule :

où $R_7$ et $R_8$ identiques ou différents représentent chacun un atome d'hydrogène

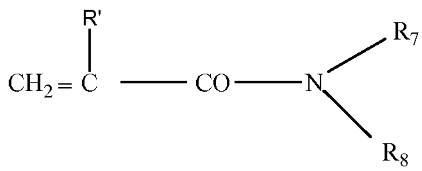

ou un groupe alkyle en $C_1$ à $C_{12}$ linéaire ou ramifié, ou $R_7$ représente H et $R_8$ représente un groupement

1,1-diméthyl-3-oxobutyl,
et R' désigne H ou méthyle.
et **en ce que** le ou lesdits deuxièmes monomères, qui sont tels que l'homopolymère préparé à partir de ces monomères a une températures de transition vitreuse inférieure ou égale à 20°C, sont choisis parmi :

- les acrylates de formule $CH_2 = CHCOOR_3$,
$R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,
- les méthacrylates de formule $CH_2 = C(CH_3)\text{-}COOR_4$,
$R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5\text{-}CO\text{-}O\text{-}CH = CH_2$
où $R_5$ représente un groupe alkyle en $C_4$ à $C_{12}$ linéaire ou ramifié ;
- les éthers d'alcool vinylique et d'alcool en $C_4$ à $C_{12}$,
- les N-alkyl en $C_4$ à $C_{12}$ acrylamides, tels que le N-octylacrylamide,
- et leurs mélanges.

**14.** Composition selon la revendication précédente, **caractérisée en ce que** ledit copolymère séquencé est tel que ladite première séquence est obtenue à partir d'au moins un monomère acrylate de formule $CH_2 = CH\text{-}COOR_2$ dans laquelle $R_2$ représente un groupe cycloalkyle $C_4$ à $C_{12}$ et d'au moins un monomère méthacrylate de formule $CH_2 = C(CH_3)\text{-}COOR'_2$ dans laquelle $R'_2$ représente un groupe cycloalkyle $C_4$ à $C_{12}$, et ladite deuxième séquence étant obtenue à partir d'au moins un deuxième monomère température de transition vitreuse inférieure ou égale à 20°C et d'un monomère additionnel, de préférence l'acide acrylique.

**15.** Composition selon l'une quelconques des revendications 13 et 14, **caractérisée en ce que** $R_2$ et $R'_2$ représentent indépendamment ou simultanément un groupe isobornyle.

**16.** Composition selon l'une quelconque des revendications précédentes, **caractérisée en ce que** ledit copolymère éthylénique séquencé comprend de 50 à 80% en poids de méthacrylate/acrylate d'isobornyle, de 10 à 30% en poids d'acrylate d'isobutyle et de 2 à 10% en poids d'acide acrylique.

**17.** Composition selon l'une quelconque des revendications précédentes dans laquelle le ou les solvants volatils sont choisis parmi l'eau et les solvants organiques non siliconés et siliconés.

**18.** Composition selon l'une quelconque des revendications précédentes comprenant un ou plusieurs composés siliconés additionnels choisis parmi les polysiloxanes présentant une viscosité supérieure à 100 cst, de préférence choisis parmi les huiles de silicone et les résines de silicone, encore plus particulièrement choisis parmi les huiles de type polydiméthylsiloxanes. et les résines de silicones.

**19.** Composition selon l'une quelconque des revendications comprenant un ou plusieurs pigments.

**20.** Procédé de traitement des fibres kératiniques comprenant l'application sur les fibres kératiniques d'une composition telle que définie à l'une quelconque des revendications 1 à 19, éventuellement suivie d'un séchage à une température supérieure à 40°C.

Europäisches Patentamt
European Patent Office
Office européen des brevets

# RAPPORT DE RECHERCHE EUROPEENNE

Numéro de la demande
EP 10 16 6438

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | FR 2 894 813 A1 (OREAL [FR]) 22 juin 2007 (2007-06-22)<br>* page 1, ligne 42 - page 2, ligne 14; revendications 1,24,25,61-67 *<br>* page 3, ligne 19 - page 21, ligne 21 *<br>* page 39, ligne 40 - page 45, ligne 28 *<br>* exemples 5,6 *<br>----- | 1-20 | INV.<br>A61K8/81<br>A61K8/90<br>A61Q5/06<br>A61Q1/00 |
| Y | FR 2 825 628 A1 (OREAL [FR]) 13 décembre 2002 (2002-12-13)<br>* page 2, ligne 2 - ligne 13 *<br>* page 3, ligne 27 - page 5, ligne 18 *<br>* page 6, ligne 21 - page 25, ligne 28; revendications 1,921,22 *<br>----- | 1-20 | |
| Y | FR 2 864 897 A1 (OREAL [FR]) 15 juillet 2005 (2005-07-15)<br>* page 3, ligne 1 - page 13, ligne 2 *<br>* page 16, ligne 24 - page 21, ligne 31 *<br>* revendications; exemples 1-13 *<br>----- | 1-20 | DOMAINES TECHNIQUES RECHERCHES (IPC) |
| Y | WO 02/34218 A2 (3M INNOVATIVE PROPERTIES CO [US]) 2 mai 2002 (2002-05-02)<br>* page 2, ligne 30 - page 5, ligne 30 *<br>* page 7, ligne 12 - page 8, ligne 7 *<br>* exemples *<br>----- | 1-20 | A61K<br>A61Q |

Le présent rapport a été établi pour toutes les revendications

| Lieu de la recherche | Date d'achèvement de la recherche | Examinateur |
|---|---|---|
| Munich | 8 octobre 2010 | Loloiu, Teodora |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................................
& : membre de la même famille, document correspondant

1

EPO FORM 1503 03.82 (P04C02)

## ANNEXE AU RAPPORT DE RECHERCHE EUROPEENNE RELATIF A LA DEMANDE DE BREVET EUROPEEN NO. EP 10 16 6438

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

08-10-2010

| Document brevet cité au rapport de recherche | Date de publication | Membre(s) de la famille de brevet(s) | Date de publication |
|---|---|---|---|
| FR 2894813 A1 | 22-06-2007 | EP 1797868 A1 | 20-06-2007 |
| | | JP 2007161716 A | 28-06-2007 |
| FR 2825628 A1 | 13-12-2002 | AT 372110 T | 15-09-2007 |
| | | DE 60222255 T2 | 29-05-2008 |
| | | EP 1392222 A1 | 03-03-2004 |
| | | ES 2292780 T3 | 16-03-2008 |
| | | WO 02098377 A1 | 12-12-2002 |
| | | JP 3951000 B2 | 01-08-2007 |
| | | JP 2005520777 T | 14-07-2005 |
| | | PT 1392222 E | 30-11-2007 |
| | | US 2004161394 A1 | 19-08-2004 |
| | | US 2010247466 A1 | 30-09-2010 |
| FR 2864897 A1 | 15-07-2005 | WO 2005067871 A1 | 28-07-2005 |
| WO 0234218 A2 | 02-05-2002 | AT 385772 T | 15-03-2008 |
| | | AU 9303801 A | 06-05-2002 |
| | | AU 2001293038 B2 | 17-08-2006 |
| | | BR 0114845 A | 07-10-2003 |
| | | CA 2426683 A1 | 02-05-2002 |
| | | CN 1535133 A | 06-10-2004 |
| | | EP 1370219 A2 | 17-12-2003 |
| | | ES 2300365 T3 | 16-06-2008 |
| | | JP 2004512292 T | 22-04-2004 |
| | | MX PA03003570 A | 14-10-2003 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 1392222 A
- EP 1435900 A
- FR 2782723
- US 5246694 A
- WO 2005075567 A
- US 20040180011 A
- US 5266321 A
- US 4742142 A
- US 5654362 A
- FR 2864784
- US 4970252 A
- US 4987169 A
- US 5412004 A
- US 5760116 A
- JP 61194009 A
- US 5236986 A
- US 5837793 A
- US 5811487 A
- US 5538793 A
- US 4693935 A
- US 4728571 A
- US 4972037 A
- EP 0412704 A
- EP 0412707 A
- EP 0640105 A
- WO 9500578 A
- FR 2679771
- EP 1184426 A
- DE 10238090
- US 4578266 A
- EP 1400234 A
- EP 708114 A
- WO 02056847 A
- WO 0247619 A
- US 5783657 A
- EP 1266647 A
- FR 0216039
- WO 03105788 A
- US 2002005562 A
- US 5221534 A

### Littérature non-brevet citée dans la description

- Polymer Handbook. John Wiley, 1989
- *Cosmetics and Toiletries,* Février 1990, vol. 105, 53-64
- **S. Caillère ; S. Hénin ; M. Rautureau.** Masson. 1982
- Silica silylate. CTFA. 1995